# EUROPEAN PATENT APPLICATION

(11) **EP 2 808 013 A1**
(43) Date of publication of application: **03.12.2014**
(21) Application number: 14178298.7
(22) Date of filing: 25.11.2009
(51) Int. Cl.: A61K 9/20, A61K 31/138, A61K 31/4166, A61K 31/444, A61K 31/513, A61K 31/519, A61K 47/10, A61K 47/32, A61K 47/38, A61K 9/00

(54) **Orally rapidly disintegrating tablet and process for producing same**

(30) Priority: 25.11.2008 JP 2008299017
(62) Divisional of application: 09829094.3
(71) Applicant: Mitsubishi Tanabe Pharma Corporation, Osaka 541-8505 (JP)
(72) Inventor: Sugimoto, Masaaki, Osaka, 541-8505 (JP); Kitaoka, Kenichi, Osaka, 541-8505 (JP); Saito, Yasunori, Osaka, 541-8505 (JP); Uemura, Katsuji, Osaka, 541-8505 (JP)
(74) Representative: Hoffmann Eitle

(57) **Abstract**

Disclosed is an orally rapidly disintegrating tablet characterized in that the tablet can be produced in a conventional tablet manufacturing facility and has a satisfactory level of hardness for practical applications, and the change in properties of the tablet (i.e., decreased in hardness of the tablet, and delay of the disintegration time of the tablet in the oral cavity) are rarely caused by factors such as humidity. The orally rapidly disintegrating tablet has hardness of 40N or more, can be disintegrated in the oral cavity within 60 seconds, and is produced by compressing of a mixture of (a) an active ingredient, (b) an excipient having good water wettability, (c) a water-insoluble polymer that is well compactible and does not substantially cause a decrease in the water wettability of the excipient and (d) a disintegrating agent.

## Description

### TECHNICAL FIELD

The present invention can be prepared in conventional tablet manufacturing facilities, and relates to a novel orally rapidly disintegrating tablet having a practical formulation strength in each step including preparation, distribution and formulation as well as a rapid disintegration property in the oral cavity, and to a process for preparation thereof.

### BACKGROUND ART

Generally, orally rapidly disintegrating tablets in medical care have been highly required in patients sufferring from Parkinson's diseases, Alzheimer's diseases or dysphagia as well as postsurgical patients who are difficult to straighten up. Various orally rapidly disintegrating tablets including formulations prepared by freeze-drying have been developed and launched until now, while research developments of orally rapidly disintegrating tablets which can be prepared in conventional facilities without any special formulation components or any manufacturing facilities have recently become the mainstream in view of production costs, etc.

Orally rapidly disintegrating tablets are needed to show rapidly disintegrating properties in the oral cavity as well as to have so sufficient physical strength (i.e., hardness) as not to be destroyed during each step including preparation, distribution and formulation and handling including taking out the tablets from blister package. Such tablets often contain fresh sugar alcohols (including mannitol, erythritol) as an excipient in view of comfortable dosing. For example, WO97/047287 pamphlet (Patent Document 1) discloses orally rapidly disintegrating tablets obtained by compressing a mixture comprising sugar alcohols or sugars in the average particle size of 30 µm or below, an active ingredient and a disintegrating agent. However, there have been drawbacks that micronized mannitol used as a formulation component has caused tableting defects such as adhesion to die and punch and lowering flowability of a mixture for tableting.

JP-A-2006-70046 (Patent Document 2) discloses orally rapidly disintegrating tablets obtained by compressing a mixture comprising sugar alcohols or sugars in the average particle size of 30 to 300 µm, an active ingredient, a disintegrating agent and celluloses, and JP-A-2000-119175 (Patent Document 3) discloses orally rapidly disintegrating tablets obtained by compressing a mixture comprising an active ingredient, crystalline cellulose, lactose, agar powders and low-substituted hydroxypropylcellulose or hydroxypropyl starch. These tablets have some drawbacks such as bad feelings on the tongue in dosing due to comparatively large contained amounts of water-insoluble components in the tablet.

JP-A-2000-44490 (Patent Document 4) discloses rapidly disintegrating tablets wherein methacrylic acid copolymer type C, which is described in US Pharmacopeia (USP/NF), is compounded as a disintegrating agent or a disintegrating auxiliary. However, this tablet is typically prepared by direct tableting, and polymers used therein are limited to methacrylic acid copolymer type C (Eudragit® L100-55 or L30D-55).

JP-A-2004-315483 (Patent Document 5) and JP-A-2006-199632 (Patent Document 6) disclose orally rapidly disintegrating tablets obtained by compressing a granule comprising a mixture comprising sugars or sugar alcohols and water-insoluble hydrophilic components. In these tablets, trehalose, lactose or mannitol (or a mixture thereof) is compounded as an excipient, and starch, cereal flour containing starch, microparticulate silicic anhydride, hydroxypropyl starch or crospovidone (or a mixture thereof) is compounded as a water-insoluble hydrophilic component. However, there have been drawbacks that the hardness of tablet has tended to be decreased during storage due to high hygroscopicities of the water-insoluble hydrophilic component. Actually, Patent Document 5 discloses test results which show that the hardness of tablet is decreased to about 1/2 under humidified storage conditions (25°C/75% RH).

The 2nd PLCM symposium lecture summary, page 27 (Nonpatent Document 1) discloses granules obtained by spray-granulating a water-insoluble substance Kollicoat SR30D (which is a water dispersion of vinyl acetate) to a mixture of mannitol and crospovidone as an excipient appropriate to the preparation of orally rapidly disintegrating tablets by a direct tableting. However, it does not disclose any water-insoluble substances except for Kollicoat SR30D.

Recently, multiple solid formulations have often been packaged in a single unit by using an automatic tablet packaging machine in dispensing pharmacies. Such a one dose packaging has the advantage that mis-dosings of medications by patients can be avoided and the drug compliance can be improved. On the other hand, in the one dose packaging by the automatic tablet packaging machine, a formulation has been required to have so sufficient hardness as not to be destroyed in the packaging operation. In this respect, it has been also reported that the orally rapidly disintegrating tablet wherein the hardness of tablet (kg) divided by the tablet weights (mg) is 0.022 or above can be operated by the automatic tablet packaging machine (Nonpatent Document 2: PHARM TECH JAPAN Vol.22 No.3 (2006)).

Since the above one dose packaging results in an exposure of a formulation to the atmosphere for a given period of time from the release of the formulation from blister package to the one dose packaging of the formulation, some changes of formulation properties, etc. by moisture absorption are a concern. Simultaneously, since packaging materials after the one dose packaging have not so much exclusion of moisture compared to blister package, significant deteriorations of the formulation properties (particularly, the hardness of tablet) before dosing are also a concern in a hygroscopic formulation. Therefore, there has been a need for orally rapidly disintegrating tablets having a property that the formulation properties (i.e., rapidly disintegrating property in the oral cavity and hardness) can be substantially maintained for a given period of time after the release of the formulation from blister package (i.e., during one doese packaging operations and for a given period of time from one dose packaging to dosing).

Under the circumstance, there has been a need for the development of orally rapidly disintegrating tablets which can be prepared using conventional tablet manufacturing facilities and of which the formulation properties (i.e., hardness and rapidly disintegrating property in the oral cavity of the tablet) can be substantially maintained in each step including manufacturing, distribution and dispensing.
[Patent Document 1] WO97/047287 pamphlet
[Patent Document 2] JP-A-2006-70046
[Patent Document 3] JP-A-2000-119175
[Patent Document 4] JP-A-2000-44490
[Patent Document 5] JP-A-2004-315483
[Patent Document 6] JP-A-2006-199632

[Nonpatent Document 1] 2nd PLCM Symposium Lecture Summary (Kouen Youshi), page 27
[Nonpatent Document 2] PHARM TECH JAPAN Vol.22 No.3 (2006)

### DISCLOSURE OF INVENTION

### [Problems to be Resolved by Invention]

In accordance with extensive studies for solving the problems, the inventors have found that orally rapidly disintegrating tablets can be obtained by combining as formulation components an excipient having good water wettability (including sugar alcohol) and a water-insoluble polymer that is well compactible and does not substantially cause a decrease in the water wettability of the excipient, which the orally rapidly disintegrating tablets can be prepared using conventional tablet manufacturing facilities, have practically sufficient hardness and have the feature that said tablets are less subject to the changes of the properties by factors including moisture, i.e. a lowered hardness of the tablet and a delayed disintegration time of the tablet in the oral cavity, and have achieved the present invention.

### [Means of Solving the Problems]

The present invention is directed to:
(1) An orally rapidly disintegrating tablet, which is produced by compressing a mixture comprising (a) an active ingredient; (b) an excipient having good water wettability; (c) a water-insoluble polymer that is well compactible and does not substantially cause a decrease in the water wettability of the excipient; and (d) a disintegrating agent; and wherein a disintegration time in the oral cavity is within 60 seconds;
(2) The orally rapidly disintegrating tablet of (1), wherein the excipient having good water wettability is one or more sugar alcohols selected from the group consisting of mannitol, erythritol and xylitol; and the water-insoluble polymer that is well compactible and does not substantially cause a decrease in the water wettability of the excipient is one or more water-insoluble polymers selected from the group consisting of hydroxypropylmethylcellulose acetate succinate, ethylcellulose, hydroxypropylmethylcellulose phthalate, carboxymethylethylcellulose, methacrylic acid copolymer L, methacrylic acid copolymer S and amino methacrylate copolymer;
(3) The orally rapidly disintegrating tablet of either (1) or (2), wherein the contained amounts of the excipient having good water wettability are 30 to 95 parts by weight; the contained amounts of the water-insoluble polymer that is well compactible and does not substantially cause a decrease in the water wettability of the excipient are 1 to 10 parts by weight; and the contained amounts of the disintegrating agent are 1 to 15 parts by weight, in 100 parts by weight of the tablet;
(4) The orally rapidly disintegrating tablet of (3), wherein the tablet strength is 100 to 300N/cm²;
(5) The orally rapidly disintegrating tablet of (3), wherein the tablet strength is 110 to 300N/cm²;
(6) The orally rapidly disintegrating tablet of (3), wherein the tablet strength is 120 to 300N/cm²;
(7) The orally rapidly disintegrating tablet of any one of (4), (5) or (6), wherein the disintegration time in the oral cavity is 5 to 45 seconds;
(8) The orally rapidly disintegrating tablet of any one of (4), (5) or (6), wherein the disintegration time in the oral cavity is 5 to 30 seconds;
(9) The orally rapidly disintegrating tablet of any one of (4), (5) or (6), wherein the disintegration time in the oral cavity is 5 to 20 seconds;
(10) The orally rapidly disintegrating tablet of any one of (4), (5) or (6), wherein the ratio of the hardness to the tablet weights is 0.2N/mg or more;
(11) The orally rapidly disintegrating tablet of any one of (4), (5) or (6), wherein the ratio of the hardness to the tablet weights is 0.3N/mg or more;
(12) The orally rapidly disintegrating tablet of any one of (4), (5) or (6), wherein the active ingredient is nicergoline, imidapril hydrochloride, bisoprolol fumarate, taltirelin hydrate, allopurinol or avanafil;
(13) The orally rapidly disintegrating tablet of (12), wherein the active ingredient is nicergoline, imidapril hydrochloride, bisoprolol fumarate or taltirelin hydrate, and the contained amounts of the active ingredient are 0.1 to 70 parts by weight to 100 parts by weight of the tablet;
(14) The orally rapidly disintegrating tablet of (12), wherein the active ingredient is nicergoline, imidapril hydrochloride, bisoprolol fumarate or taltirelin hydrate, and the contained amounts of the active ingredient is 0.5 to 20 parts by weight to 100 parts by weight of the tablet;
(15) The orally rapidly disintegrating tablet of (12), wherein the active ingredient is nicergoline, imidapril hydrochloride, bisoprolol fumarate or taltirelin hydrate, and the contained amounts of the active ingredient is 1 to 15 parts by weight to 100 parts by weight of the tablet;
(16) The orally rapidly disintegrating tablet of (12), wherein the active ingredient is nicergoline, imidapril hydrochloride, bisoprolol fumarate or taltirelin hydrate, and the contained amounts of the active ingredient is 1 to 10 parts by weight to 100 parts by weight of the tablet;
(17) The orally rapidly disintegrating tablet of (12), wherein the active ingredient is allopurinol or avanafil, and the contained amounts of the active ingredient is 10 to 50 parts by weight to 100 parts by weight of the tablet;
(18) An orally rapidly disintegrating tablet, which is produced by compressing a mixture of (a) an active ingredient selected from the group consisting of nicergoline, imidapril hydrochloride, bisoprolol fumarate, taltirelin hydrate, allopurinol and avanafil; (b) one or more excipients selected from the group consisting of mannitol, erythritol and xylitol; (c) one or more water-insoluble polymers selected from the group consisting of hydroxypropylmethylcellulose acetate succinate, ethylcellulose, hydroxypropylmethylcellulose phthalate, carboxymethylethylcellulose, methacrylic acid copolymer L, methacrylic acid copolymer S and amino methacrylate copolymer; (d) one or more disintegrating agents selected from the group consisting of carboxymethylcellulose calcium, low-substituted hydroxypropylcellulose, carboxymethylcellulose, cross-linked carboxymethylcellulose sodium, crystalline cellulose, corn starch, pregelatinized starch, carboxymethyl starch sodium and cross-linked polyvinylpyrrolidone; and (e) a lubricant, wherein the mixture can contain one or more additives selected from the group consisting of binders, plasticizers, coating agents, deflocculating agents, solubilizers, sweeteners, acidulants, flavoring substances, pH adjusters, solubilizing agents, colorants and flavoring agents; and wherein a disintegration time in the oral cavity is within 60 seconds;
(19) The orally rapidly disintegrating tablet of (18), wherein the contained amounts of the excipient are 30 to 95 parts by weight, the contained amounts of the water-insoluble polymer are 1 to 10 parts by weight, the contained amounts of the disintegrating agent are 1 to 15 parts by weight, and the contained amounts of the lubricant are 0.01 to 3 parts by weight, in 100 parts by weight of the tablet;
(20) The orally rapidly disintegrating tablet of (19), wherein the tablet strength is 100 to 300N/cm²;
(21) The orally rapidly disintegrating tablet of (19), wherein the tablet strength is 110 to 300N/cm²;
(22) The orally rapidly disintegrating tablet of (19), wherein the tablet strength is 120 to 300N/cm²;
(23) The orally rapidly disintegrating tablet of any one of (20), (21) or (22), wherein the disintegration time in the oral cavity is 5 to 45 seconds;
(24) The orally rapidly disintegrating tablet of any one of (20), (21) or (22), wherein the disintegration time in the oral cavity is 5 to 30 seconds;
(25) The orally rapidly disintegrating tablet of any one of (20), (21) or (22), wherein the disintegration time in the oral cavity is 5 to 20 seconds;
(26) The orally rapidly disintegrating tablet of any one of (20), (21) or (22), wherein the ratio of the hardness to the tablet weights is 0.2N/mg or more;
(27) The orally rapidly disintegrating tablet of any one of (20), (21) or (22), wherein the ratio of the hardness to the tablet weights is 0.3N/mg or more;
(28) The orally rapidly disintegrating tablet of any one of (20), (21) or (22), wherein the excipient is mannitol, and the water-insoluble polymer is hydroxypropylmethylcellulose acetate succinate, hydroxypropylmethylcellulose phthalate or carboxymethylethylcellulose;
(29) A process for preparing an orally rapidly disintegrating tablet wherein a disintegration time in the oral cavity is within 60 seconds, comprising i) preparing a mixture of (a) an active ingredient; (b) an excipient having good water wettability; (c) a water-insoluble polymer that is well compactible and does not substantially cause a decrease in the water wettability of the excipient; and (d) a disintegrating agent, wherein the mixture can contain one or more additives selected from the group consisting of lubricants, binders, plasticizers, coating agents, deflocculating agents, solubilizers, sweeteners, acidulants, flavoring substances, pH adjusters, solubilizing agents, colorants and flavoring agents, followed by ii) compressing the mixture obtained in the above i);
(30) The process of (29), wherein the active ingredient is nicergoline, imidapril hydrochloride, bisoprolol fumarate, taltirelin hydrate, allopurinol or avanafil; the excipient having good water wettability is mannitol, erythritol or xylitol; the water-insoluble polymer that is well compactible and does not substantially cause a decrease in the water wettability of the excipient is one or more water-insoluble polymers selected from the group consisting of hydroxypropylmethylcellulose acetate succinate, ethylcellulose, hydroxypropylmethylcellulose phthalate, carboxymethylethylcellulose, methacrylic acid copolymer L, methacrylic acid copolymer S and amino methacrylate copolymer; and the disintegrating agent is carboxymethylcellulose calcium, carboxymethylcellulose, cross-linked carboxymethylcellulose sodium, carboxymethyl starch sodium or cross-linked polyvinylpyrrolidone;
(31) The process of (30), wherein the tablet strength of the orally rapidly disintegrating tablet is 100 to 300N/cm²; and
(32) The orally rapidly disintegrating tablet of either (1) or (18), wherein the diameter is 5 to 10 mm, the weight is 100 to 300 mg, and the hardness is 15N to 90N.

### BEST MODE FOR CARRYING OUT THE INVENTION

The active ingredient used in the present invention includes any orally-available drugs, and the typical active ingredient includes vitamins (including vitamin A, vitamin B₁, vitamin B₆, vitamin C, vitamin D, vitamin E), antipyretic, analgesic and antiphlogistic (including aspirin, etenzamide, acetaminophen, ibuprofen), antipsychotic agents (including chlorpromazine, reserpine), agents for central nervus system (including taltirelin hydrate), antidepressants (including imipramine), hypnosedatives (including diazepam, nitrazepam, quazepam), antispasmodic agents (including scopolamine hydrobromide), improving agents for cerebral metabolism (including meclofenoxate hydrochloride), improving agents for cerebral circulation (including vinpocetine, nicergoline), antiepileptic agents (including phenytoin, carbamazepine, sodium valproate), sympathomimetic agents (including isoproterenol hydrochloride), antiulcer agents (including omeprazole, lansoprazole, famotidine, cimetidine), bronchodilators (including theophylline, trimetoquinol hydrochloride), antitussive drugs (including tipepidine hibenzate), antiallergic agents (including seratrodast, pemirolast potassium), antihistamine agents (including diphenhydramine hydrochloride), cardiotonic agents (including caffeine), antiarrhythmic agents (including propranolol hydrochloride), diuretic agents (including hydrochlorothiazide), antihypertensive agents (including calcium antagonistic agents such as diltiazem hydrochloride, amlodipine or nifedipine; ACE inhibitors such as imidapril hydrochloride, quinapril hydrochloride or enalapril; angiotensin II receptor antagonists such as candesartan cilexetil or losartan potassium; β receptor blocking agents such as betaxolol hydrochloride or bisoprolol fumarate), coronary vasodilators (including verapamil hydrochloride), peripheral vasodilators (including cinnarizine), anti-hyperlipidemic agents (including HMG-CoA reductase inhibitors such as pravastatin, fluvastatin sodium, cerivastatin, simvastatin or atorvastatin calcium), antidiabetic agents (including tolbutamide, voglibose, glybenclamide), antihyperuricemic agents (including allopurinol), therapeutic agents for erectile dysfunction (including PDE5 inhibitors such as sildenafil citrate, baldenafil, avanafil or tadalafil), antirheumatic agents (including methotrexate), but is not limited thereto. Among them, imidapril hydrochloride, bisoprolol fumarate, nicergoline, taltirelin hydrate, avanafil or allopurinol is particularly preferable.

Tha dosage of the active ingredient depends on the type of the active ingredient and can generally include 0.1 to 70 parts by weight, preferably 0.5 to 50 parts by weight, more preferably 1 to 30 parts by weight, to 100 parts by weight of the tablet, but is not limited thereto. More particularly, the contained amounts of each active ingredient into a tablet are preferably determined so as to contain therapeutically effective amounts thereof per a tablet depending on dosage and administration. For example, when the active ingredient is imidapril hydrochloride, bisoprolol fumarate, nicergoline or taltirelin hydrate, the dosage of the active ingredient can be 0.1 to 70 parts by weight, preferably 0.5 to 20 parts by weight, more preferably 1 to 15 parts by weight, particularly preferably 1 to 10 parts by weight, to 100 parts by weight of the tablet. When the active ingredient is avanafil or allopurinol, the dosage of the active ingredient can be 10 to 50 parts by weight, preferably 10 to 40 parts by weight, more preferably 10 to 30 parts by weight, to 100 parts by weight of the tablet.

The excipient "having good water wettability" in the present invention is defined as follows. Specifically, if the wetness time is within 300 seconds in measuring the wetness time in water of a tablet which is obtained by compressing an excipient at 1000 kg of a tableting pressure using a flat punch of 10 mm in diameter in accordance with the method of the following Experimental Example 1, said excipient is the excipient "having good water wettability".

The excipient having good water wettability includes one or more sugars or sugar alcohols selected from the group consisting of mannitol, erythritol and xylitol, for example. Among them, mannitol is preferable. The average particle size of the excipient is usually 0.1 to 500 µm, preferably 0.5 to 300 µm, particularly preferably 1 to 100 µm, but is not limited thereto. The excipient as described above can be contained solely, or two or more excipients as described above can be contained in combination into the orally rapidly disintegrating tablet of the present invention. The contained amounts of the excipient can be 30 to 95 parts by weight, preferably 40 to 95 parts by weight, to 100 parts by weight of the tablet.

The water-insoluble polymer "that is well compactible" and "does not substantially cause a decrease in the water wettability of the excipient" in the present invention refers to a water-insoluble polymer having the properties defined in the following both (1) and (2).

(1) To a mixture comprising 97 parts by weight of an excipient and 3 parts by weight of a water-insoluble polymer is added 5 parts by weight of aqueous ethanol solution (80% W/W), and the mixture is kneaded and then dried to give a granule. The obtained granule (300 mg) is compressed (a flat punch with 10 mm of diameter, a tableting pressure: 1000 kg) to give a molded form (i.e., tablet). When the form has three or more times of hardness compared to a molded form (i.e., tablet) which is similarly obtained by compressing the same excipient only, the water-insoluble polymer is the water-insoluble polymer "that is well compactible".

(2) If the wetness time is within 300 seconds in measuring the wetness time in water of the tablet prepared by the above (1) comprising the excipient and the water-insoluble polymer in accordance with the method of the following Experimental Example 1, said water-insoluble polymer is the water-insoluble polymer "that does not substantially cause a decrease in the water wettability of the excipient".

The water-insoluble polymer that is well compactible and does not substantially cause a decrease in the water wettability of the excipient can be any polymers having said property without any limitation, and includes an enteric polymer such as hydroxypropylmethylcellulose acetate succinate (e.g., HPMC-AS/Shin-Etsu Chemical Co., Ltd.), ethylcellulose (e.g., ETHOCEL®/The Dow Chemical Company), methacrylic acid copolymer L (e.g., Eudragit L/Roehm), methacrylic acid copolymer S (e.g., Eudragit S/Roehm), hydroxypropylmethylcellulose phthalate (e.g., HP-50/Shin-Etsu Chemical Co., Ltd.) or carboxymethylethylcellulose (e.g., CMEC/Sanyo Chemical Industries, Ltd.), or amino methacrylate copolymer (e.g., Eudragit RS or RL/Roehm). Among them, hydroxypropylmethylcellulose acetate succinate or hydroxypropylmethylcellulose phthalate is preferable. The average particle size of the water-insoluble polymer is usually 0.1 to 500 µm, preferably 0.5 to 300 µm, particularly preferably 1 to 100 µm of the water-insoluble polymer without any limitation.

The water-insoluble polymer as described above can be contaiend solely, or two or more water-insoluble polymers as described above can be contained in combination into the orally rapidly disintegrating tablet of the present invention. The contained amounts of the water-insoluble polymer can be 1 to 10 parts by weight, preferably 3 to 10 parts by weight, to 100 parts by weight of the tablet.

The disintegrating agent includes celluloses such as carboxymethylcellulose calcium, low-substituted hydroxypropylcellulose, carboxymethylcellulose, cross-linked carboxymethylcellulose sodium or crystalline cellulose, starches such as corn starch, pregelatinized starch or carboxymethyl starch sodium, cross-linked polyvinylpyrrolidone, etc. The contained amounts of the disintegrating agent are usually 1 to 15 parts by weight, preferably 3 to 10 parts by weight, to 100 parts by weight of the tablet.

The lubricant includes magnesium stearate, calcium stearate, stearic acid, sucrose fatty acid ester, talc, polyethyleneglycol, etc. The contained amounts of the lubricant can be 0.01 to 3 parts by weight, preferably 0.01 to 2 parts by weight, more preferably 0.01 to 1 parts by weight, to 100 parts by weight of the tablet.

The orally rapidly disintegrating tablet of the present invention can optionally contain additional additives in appropriate amounts depending on the purpose of compounding in addition to the above components. Such additives include binders, plasticizers, coating agents, deflocculating agents, solubilizers, sweeteners, acidulants, flavoring substances, pH adjusters, solubilizing agents, colorants, flavoring agents, etc. The binders include sodium alginate, gelatin, dextrin, hydroxyethylcellulose, hydroxypropylcellulose, methylcellulose, polyvinyl alcohol, polyvinylpyrrolidone, etc. The plasticizers include triethyl citrate, glycerin fatty acid ester, polyethyleneglycol, etc. The coating agents include ethylcellulose, hydroxypropylmethylcellulose, etc. The deflocculating agents include talc, calcium stearate, etc. The solubilizers include sucrose fatty acid ester, sorbitan monostearate, sodium lauryl sulfate, etc. The sweeteners include aspartame, saccharin, dipotassium glycyrrhizate, stevia, etc. The acidulants (organic acids) include citric acid, malic acid, ascorbic acid, fumaric acid, etc. The flavoring substances include 1-menthol, sodium chloride, acesulfame potassium, sucralose, etc. The pH adjusters include citrate, phosphate, carbonate, acetate, etc. The solubilizing agents include cyclodextrin, arginine, lysine, tris-aminomethane, etc. The colorants include yellow iron sesquioxide, iron sesquioxide, sodium copper chlorophyllin, etc. The flavoring agents include orange oil, lemon oil, mint oil, eucalyptus oil, etc.

The orally rapidly disintegrating tablet of the present invention can be prepared in accordance with conventional methods in the tablet production, and for example, it can be prepared by i) preparing a mixture of (a) an active ingredient; (b) an excipient having good water wettability; (c) a water-insoluble polymer that is well compactible and does not substantially cause a decrease in the water wettability of the excipient; and (d) a disintegrating agent, wherein the mixture can contain one or more additives selected from the group consisting of lubricants, binders, plasticizers, coating agents, deflocculating agents, solubilizers, sweeteners, acidulants, flavoring substances, pH adjusters, solubilizing agents, colorants and flavoring agents, followed by ii) compressing the mixture obtained in the above i).

The mixture of each of the above components (a) to (d) and additional additives can be obtained by sequentially mixing powders of each component. The mixture can be also obtained by granulating any components (e.g., an excipient and a water-insoluble polymer, or an active ingredient, an excipient and a water-insoluble polymer) by a conventional granulation method to combine the obtained granules with the remaining components (i.e., additives such as disintegrating agents, lubricants, sweeteners, acidulants). Each of the components (a) to (d) or the additional additives can be optionally precoated by conventional methods using watersoluble or water-insoluble film-forming polymers, etc.

The process for mixing the above components can be carried out in accordance with the conventional method using a double cone blender (e.g., double cone mixer/manufactured by Yashimakakoki Co., Ltd.), a fluid-bed granulator (multiplex/manufactured by Powrex Corporation, SPIR-A-FLOW/manufactured by FREUND CORPORATION), a high speed mixer granulator (high speed mixer/manufactured by Fukae Powtec., a vertical granulator/manufactured by Powrex Corporation), a vibration sifter (vibration sifter manufactured by Maldalton Co., Ltd.), etc., but is not limited thereto.

As a process for granulation of the above components, a dry granulation, a wet granulation, etc. can be used, for example. In the dry granulation, a powdery mixture of the above components (a) to (d) can be granulated by using a roller compactor, a roll granulator, etc. to give the desired granule. In the wet granulation, a granulation can be carried out by using a fluid-bed granulator, a high speed mixer granulator, etc. with adding a solution (e.g., ethanol solution, ethanol/aqueous solution) or an aqueous dispersion of a water-insoluble polymer (c) to a powdery mixture of the active ingredient (a) and an excipient (b) under flow by means of spray, etc., and then the resulted granule can be dried to give the desired granule which is suitable for a subsequent compressing.

The granulated granule resulted in this way can be mixed with various additives (e.g., one or more additives selected from the group consisting of disintegrating agents, lubricants, solubilizers, sweeteners, acidulants, flavoring substances, pH adjusters, solubilizing agents, colorants and flavoring agents) before the compressing, if needed.

A compressing of the mixture of the components (a) to (d) resulted in this way can be carried out by using a conventional tableting machine such as a single tableting machine, a rotary tableting machine, etc. A tableting pressure can be optionally adopted depending on the targeted property of tablet (including hardness), and it can be usually 10 to 5000 kg/punch, preferably about 20 to 4000 kg/punch, particularly preferably about 100 to 2000 kg/punch of tableting pressure. In the compressing, a process comprising alternately tableting a mixture of the above components (a) to (d) and a mixture of a lubricant and a fluidizer (JP-10-298061A), a process comprising compressing with spraying lubricant powders to a tableting punch (JP-48-20103A), etc. can be adopted to prevent tableting failures including sticking.

A detail of one embodiment of the preparation of the orally rapidly disintegrating tablet in the present invention is as follows. Specifically, the orally rapidly disintegrating tablet in the present invention can be prepared by, for example,
A) adding a solution (e.g., ethanol/aqueous solution, etc.) or an aqueous dispersion of a water-insoluble polymer (c) that is well compactible and does not substantially cause a decrease in the water wettability of the excipient to a mixture of the active ingredient (a) and an excipient having good water wettability (b) to granulate and dry the granule, then mixing the resulting granule and a disintegrating agent, and if needed, any other additives (including a lubricant, a sweetener, an acidulant such as an organic acid), and compressing the mixture, or
B) adding a solution (e.g., ethanol/aqueous solution, etc.) or an aqueous dispersion of a water-insoluble polymer (c) that is well compactible and does not substantially cause a decrease in the water wettability of the excipient to an excipient (b) having good compactibility and good water wettability to granulate and dry the granule, then mixing the resulting granule and the active ingredient (a), a disintegrating agent, and if needed, any other additives (including a lubricant, a sweetener, an acidulant such as an organic acid), and compressing the mixture. In the above preparation, the active ingredient or other additives can be optionally coated with a film forming polymer such as ethylcellulose, etc.

A form of the orally rapidly disintegrating tablet of the present invention resulted in this way can be various forms including round, oval, caplet, doughnut shape, but is not limited thereto.

A preferable hardness of tablet in the orally rapidly disintegrating tablet of the present invention which is measured by a tablet hardness tester varies depending on factors including forms, sizes, weights, and for example, the hardness of tablet in a round tablet with 5 to 10 mm of diameter (weight 100 to 300 mg) is usually 15N to 90N, preferably 20N to 90N, more preferably 40N to 90N, further more preferably 50 to 90N. A tablet strength (i.e., a ratio of the hardness of tablet to rupture areas of tablet) in the orally rapidly disintegrating tablet of the present invention is usually 100N/cm² or more, preferably 110 to 300N/cm², further more preferably 120 to 300N/cm².

The disintegration time of the tablet in the oral cavity in the present invention depends on form and thickness thereof, and is usually within 60 seconds (5 to 60 seconds), preferably 5 to 45 seconds, more preferably 5 to 35 seconds, further more preferably 5 to 30 seconds, particularly preferably 20 seconds (5 to 20 seconds).

A tablet which is appropriate for an one dose packaging by using an automatic tablet packaging machine can be also easily obtained by the present invention. Said tablet is preferable for the purpose of reducing or avoiding damages of tablets during one dose packaging operations. Said tablet has, for example, 0.2N/mg or more, preferably 0.3N/mg or more, of the ratio of hardness to tablet weights, which varies depending on factors including forms, sizes, weights of tablets.

### EXAMPLES

### Experimental Example 1

A mixture (300 mg) of an excipient (97 parts by weight (or 90 parts by weight)) and a water-insoluble polymer (hydroxypropylmethylcellulose acetate succinate, HPMC-AS, grade; HF) (3 parts by weight (or 10 parts by weight)) was compressed using Compaction Analyzer (manufactured by Kikusui Seisakusho Ltd., flat punch: diameter 10 mm) at a tableting pressure of 1000 kg/punch to give a tablet. Each water wettability (wetness time) and hardness of each tablet was measured in accordance with the following Assessment Procedure, and excipients were classified into the following 2 groups on the basis of the measurement results (Table 1 as below).
Group A: Excipients having good water wettability and compactibility when a tablet comprises a water-insoluble polymer, which show that a wetness time of tablet is within 300 seconds and a hardness of tablet is over 3 times of an excipient only.
Group B: Excipients having poor water wettability or compactibility when a tablet comprises a water-insoluble polymer, which show that a wetness time of tablet is over 300 seconds or a hardness of tablet is less than 3 times of an excipient only.

### Assessment Procedure:

### a) Water wettability (wetness time) of tablet

A water wettability (wetness time) of a tablet was measured in accordance with a known procedure [Chem. Pharm. Bull. 44 (1), 2121-2127 (1996)] as below. Specifically, purified water (6 mL) was put in a weighing dish, and thereon were placed quarto tissue papers (205 mm x 120 mm, KimWipe S-200, manufactured by NIPPON PAPER CRECIA Co., LTD). The papers were entirely moistened, and then thereon was gently placed one tablet, and a wetness time required for entirely moistening the tablet was measured.

### b) Hardness of tablet

A hardness of tablet was measured using a tablet hardness tester (manufactured by Schleuniger, type: 6D). The measurement was carried out thrice, and a hardness of tablet was obtained on an average thereof.

**[Table 1]**

| Excipients | | Wetness time (seconds) | | Hardness (N) | |
|---|---|---|---|---|---|
| | | Tablet obtained by compressing an excipient only | Tablet obtained by compressing a mixture of an excipient and a water-insoluble polymer | Tablet obtained by compressing an excipient only | Tablet obtained by compressing a mixture of an excipient and a water-insoluble polymer |
| Group A | D-mannitol | 18 | 43 (*1) | 12 | 53 (*1) |
| | Erythritol | - | 37 (*2) | <4 | 23 (*2) |
| | Xylitol | 3 | 74 (*2) | 12 | 38 (*2) |
| Group B | Sorbitol | >300 | >300 (*1) | 229 | 207 (*1) |
| | Purified sucrose | 3 | >300 (*1) | 41 | 53 (*1) |
| *1: A mixture of 97 parts by weight of an excipient and 3 parts by weight of a water-insoluble polymer | | | | | |
| *2: A mixture of 90 parts by weight of an excipient and 10 parts by weight of a water-insoluble polymer | | | | | |

### Experimental Example 2

To a mixture of D-mannitol (97 parts by weight) and various water-insoluble polymers (3 parts by weight) was added aqueous 80% (W/W) ethanol solution (5 parts by weight). The mixture was kneaded in a stainless vessel and then granulated with Japanese Pharmacopoeia No. 22 mesh, and the resulting granule was dried at 50°C for 2 hours in a ventilatory box drying machine to give a granulated granule. The granulated granule (300 mg) was compressed (flat punch: diameter 10 mm, tableting pressure: 1000 kg/punch) by Compaction Analyzer (manufactured by Kikusui Seisakusho Ltd.) to give a tablet. Each water wettability (wetness time) and hardness of each tablet was measured in accordance with the Assessment Procedure of Experimental Example 1. A water-insoluble polymer was classified into the following 3 groups on the basis of the measurement results (Table 2 as below).
Group 1: Water-insoluble polymers that do not cause a decrease in the water wettability of sugar alcohol and have good compactibility, which show that a wetness time of tablet is within 300 seconds and a hardness of tablet is over 3 times of mannitol only.
Group 2: Water-insoluble polymers that do not cause a decrease in the water wettability of sugar alcohol and have poor compactibility, which show that a wetness time of tablet wherein the tablet comprises a water-insoluble polymer is within 300 seconds and a hardness of tablet is less than 3 times of mannitol only.
Group 3: Water-insoluble polymers that cause a decrease in the water wettability of sugar alcohol and have good compactibility, which show that a wetness time of tablet wherein the tablet comprises a water-insoluble polymer is over 300 seconds and a hardness of tablet is over 3 times of mannitol only.

**[Table 2]**

| Water-insoluble polymer | | | Wetness time (seconds) | Hardness (N) | Rising rates of hardness (times) |
|---|---|---|---|---|---|
| Group 1 | Hydroxypropyl methylcellulose acetate succinate | HPMC-AS/HF | 43 | 68 | 5.7 |
| | | HPMC-AS/MF | 50 | 81 | 6.8 |
| | | HPMC-AS/LF | 37 | 54 | 4.5 |
| | Ethylcellulose | EC#10 | 69 | 45 | 3.8 |
| | Hydroxypropyl methylcellulose phthalate | HP-50 | 39 | 46 | 3.8 |
| | Amino methacrylate copolymer | Eudragit RSPO | 40 | 59 | 4.9 |
| | | Eudragit RLPO | 93 | 63 | 5.3 |
| | Methacrylic acid copolymer S or L | Eudragit L100 | 66 | 61 | 5.1 |
| | | Eudragit S 100 | 20 | 48 | 4.0 |
| | Carboxymethyl ethyl cellulose (CMEC) | | 142 | 74 | 6.2 |
| Group 2 | Corn starch | | 37 | 19 | 1.6 |
| | Crospovidone | | 11 | 27 | 2.3 |
| Group 3 | Hydroxypropyl methylcellulose | TC-5EW | >300 | 70 | 5.8 |
| | Hydroxypropyl cellulose | HPC-SL | >300 | 56 | 4.7 |

### Experimental Example 3

Various excipients of Experimental Example 1 and a water-insoluble polymer (HPMC-AS/HF) were used in accordance with Examples 1 to 3 and Comparative Examples 1 to 2 to give tablets. Hardnesses of tablet were measured in accordance with the Assessment Procedure of Experimental Example 1. Disintegration times of the tablets in the oral cavity were measured as below.

### Disintegration time of tablet in the oral cavity:

One tablet each was put in three healthy male adults' mouth, and the time until the tablet was entirely disintegrated by saliva with gently putting in mouth (i.e., without biting or intensively moving a tongue, etc.) was measured to calculate the avarage value.

### Results)

Each hardness of each tablet and disintegration time of each tablet in the oral cavity was shown in the following Table 3. As seen in Table 3, in a tablet obtained using an excipient of Group A (i.e., tablets of Examples 1 to 3), each hardness was over 40N and each disintegration time of tablet in the oral cavity was 15 to 42 seconds, and each tablet showed good property as the orally disintegrating formulation. On the other hand, in a tablet obtained using an excipient of Group B (i.e., tablets of Comparative Examples 1 to 2), each hardness was over 50N, and however, each disintegration time of tablet in the oral cavity was over 60 seconds, and each tablet did not have practically sufficient property as the orally disintegrating formulation. These test results showed that excipients of Group A are preferable for the excipient to be applied to the orally disintegrating formulation of the present invention.

**[Table 3]**

| | Excipient | Water-insoluble polymer | Hardness of tablet (N) | Disintegration time (seconds) |
|---|---|---|---|---|
| Example 1 tablet | D-Mannitol | HPMC-AS/HF | 56 | 17 |
| Example 2 tablet | Erythritol | | 51 | 15 |
| Example 3 tablet | Xylitol | | 44 | 42 |
| Comparative Example 1 tablet | Sorbitol | | 57 | 129 |
| Comparative Example 2 tablet | Purified sucrose | | 53 | 65 |

### Experimental Example 4

Various water-insoluble polymers of Experimental Example 2 and an excipient (D-mannitol) were used in accordance with Examples 1, 4 to 12 and Comparative Examples 3 to 6 to give tablets. Comparative test results for hardnesses of the tablets and disintegration times of the tablets in the oral cavity were shown in the following Table 4.

As seen in Table 4, when a water-insoluble polymer of Group 1 (i.e., a water-insoluble polymer that does not cause a decrease in the water wettability of sugar alcohol and is well compactible) was used, the disintegration time in the oral cavity of a tablet having 50 to 60N hardness was within 20 seconds, and the disintegration time in the oral cavity of a tablet having 80N or more of hardness was within 22 seconds. These tablets showed good property as the orally disintegrating formulation.

On the other hand, when a water-insoluble polymer of Group 2 (i.e., corn starch or crospovidone) was used, a tablet having 80N or more of hardness could not be obtained even at 2000 kg/punch of the tableting pressure.

Further, when a water-insoluble polymer of Group 3 (i.e., hydroxypropylmethylcellulose/TC-5EW or hydroxypropylcellulose/HPC-SL) was used, the disintegration time in the oral cavity of each tablet having about 50N of hardness was over 30 seconds, and said tablet did not have a satisfied property as the orally disintegrating formulation.

The above results showed that water-insoluble polymers of Group 1 are preferable for the water-insoluble polymer to be applied to the orally disintegrating formulation of the present invention.

**[Table 4]**

| | Water-insoluble polymer (Group) | Tableting pressure | Hardness | Disintegration time |
|---|---|---|---|---|
| | | (ton/punch) | (N) | (seconds) |
| Example 1 tablet | HPMC-AS/HF (1) | 0.75 | 56 | 17 |
| | | 1.25 | 84 | 15 |
| Example 4 tablet | HPMC-AS/MF (1) | 0.70 | 61 | 15 |
| | | 1.10 | 87 | 17 |
| Example 5 tablet | HPMC-AS/LF (1) | 0.85 | 54 | 19 |
| | | 1.35 | 82 | 22 |
| Example 6 tablet | EC#10 (1) | 0.90 | 53 | 18 |
| | | 1.40 | 80 | 16 |
| Example 7 tablet | HP-50 (1) | 0.90 | 50 | 18 |
| | | 1.50 | 80 | 17 |
| Example 8 tablet | Eudragit RSPO (1) | 0.90 | 56 | 17 |
| | | 1.50 | 86 | 18 |
| Example 9 tablet | Eudragit RLPO (1) | 0.80 | 53 | 16 |
| | | 1.25 | 84 | 16 |
| Example 10 tablet | Eudragit L100 (1) | 0.80 | 51 | 16 |
| | | 1.25 | 81 | 19 |
| Example 11 tablet | Eudragit S100 (1) | 1.00 | 54 | 19 |
| | | 1.90 | 84 | 19 |
| Example 12 tablet | CMEC (1) | | | 19 |
| | | 1.10 | 83 | 20 |
| Comparative Example 3 tablet | TC-5EW (3) | 0.90 | 52 | 105 |
| Comparative Example 4 tablet | HPC-SL (3) | 0.85 | 52 | 37 |
| | | 1.35 | 81 | 46 |
| Comparative Example 5 tablet | Corn starch (2) | 2.00 | 57 | 15 |
| Comparative Example 6 tablet | Crospovidone(2) | 1.50 | 52 | 15 |
| | | 2.00 | 73 | 14 |

### Experimental Example 5

Components of the following Tables 5 to 7 were used in accordance with Examples 13 to 27 below to give orally rapidly disintegrating tablets.

Each hardness and each disintegration time in the oral cavity for each tablet was measured in accordance with the above Experimental Examples 1 and 3. The results are shown in the following Tables 8 to 10. A disintegration time of tablet in the oral cavity for each tablet was within 35 seconds, and each tablet showed good property as the orally disintegrating formulation. Further, since each value calculated by dividing a hardness by a tablet weight was over 0.216N/mg in the orally rapidly disintegrating tablets having 80N or more of hardness, it was believed that such tablets could be preferably applied to a one dose packaging by using an automatic tablet packaging machine.

**[Table 5]**

| Components | Contained amounts of each component (mg) | | | | | |
|---|---|---|---|---|---|---|
| | Example 13 | Example 14 | Example 15 | Example 16 | Example 17 | Example 18 |
| Imidapril hydrochloride | 5.0 | | | | | |
| D-Mannitol | 269.5 | | | | | |
| HPMCAS/HF | 9.0 | - | - | - | - | - |
| MPMCAS/MF | - | 9.0 | - | - | - | - |
| HPMCAS/LF | - | - | 9.0 | - | - | - |
| HP-50 | - | - | - | 9.0 | - | - |
| Eudragit RSPO | - | - | - | - | 9.0 | - |
| CMEC | - | - | - | - | - | 9.0 |
| Crospovidone | 15.0 | | | | | |
| Magnesium stearate | 1.5 | | | | | |
| Total | 300 | | | | | |

**[Table 6]**

| Components | Contained amounts of each component (mg) | | | |
|---|---|---|---|---|
| | Example 19 | Example 20 | Example 21 | Example 22 |
| Imidapril hydrochloride | 5.0 | | | |
| D-Mannitol | 269.5 | | | |
| HPMCAS/HF | 9.0 | 9.0 | 9.0 | 9.0 |
| Carboxymethylcellulose | 15.0 | - | - | - |
| Croscarmellose sodium | - | 6.0 | - | - |
| Croscarmellose calcium | - | - | 15.0 | - |
| Sodium carboxymethyl starch | - | - | - | 9.0 |
| Magnesium stearate | 1.5 | | | |
| Total | 300 | 291 | 300 | 294 |

**[Table 7]**

| Components | Contained amounts of each component (mg) | | | | |
|---|---|---|---|---|---|
| | Example 23 | Example 24 | Example 25 | Example 26 | Example 27 |
| Bisoprolol fumarate | 5.0 | 5.0 | - | - | - |
| Nicergoline | - | - | 5.0 | - | - |
| Taltirelin hydrate | - | - | - | 5.0 | - |
| Allopurinol | - | - | - | - | 100 |
| D-Mannitol | 269.5 | 269.5 | 270.15 | 236.5 | 174.5 |
| HPMCAS/HF | 9.0 | - | 8.35 | - | 9.0 |
| Eudragit L100 | - | 30.0 | - | - | - |
| Eudragit S100 | - | - | - | 27.0 | - |
| Crospovidone | 15.0 | - | 15.0 | - | 15.0 |
| Carboxymethylcellulose | - | 33.0 | - | 30.0 | - |
| Magnesium stearate | 1.5 | 1.5 | 1.5 | 1.5 | 1.5 |
| Total | 300 | 300 | 300 | 300 | 300 |

**[Table 8]**

| Tablet property | Example 13 | Example 14 | Example 15 | Example 16 | Example 17 | Example 18 |
|---|---|---|---|---|---|---|
| Tableting pressure (ton/punch) | 0.80 | 0.80 | 0.90 | 0.80 | 0.55 | 0.60 |
| Hardness (N) | 55 | 59 | 51 | 53 | 57 | 56 |
| Tablet strength (N/cm²) | 172 | 185 | 161 | 167 | 173 | 175 |
| Disintegration time (seconds) | 18 | 22 | 19 | 19 | 23 | 26 |
| Tableting pressure (ton/punch) | 1.40 | 1.40 | 1.60 | 1.40 | 0.80 | 0.95 |
| Hardness (N) | 86 | 87 | 80 | 84 | 82 | 81 |
| Tablet strength (N/cm²) | 285 | 288 | 264 | 278 | 261 | 262 |
| Hardness/weights (N/mg) | 0.287 | 0.290 | 0.267 | 0.280 | 0.273 | 0.270 |
| Disintegration time (seconds) | 20 | 22 | 26 | 23 | 20 | 27 |

**[Table 9]**

| Tablet property | Example 19 | Example 20 | Example 21 | Example 22 |
|---|---|---|---|---|
| Tableting pressure (ton/punch) | 0.80 | 0.80 | 0.80 | 0.85 |
| Hardness (N) | 50 | 52 | 50 | 53 |
| Tablet strength (N/cm²) | 157 | 163 | 157 | 166 |
| Disintegration time (seconds) | 23 | 20 | 28 | 29 |
| Tableting pressure (ton/punch) | 1.40 | 1.45 | 1.45 | 1.50 |
| Hardness (N) | 80 | 81 | 83 | 81 |
| Tablet strength (N/cm²) | 268 | 282 | 280 | 282 |
| Hardness/weights (N/mg) | 0.267 | 0.270 | 0.277 | 0.270 |
| Disintegration time (seconds) | 25 | 23 | 33 | 35 |

**[Table 10]**

| Tablet property Table property | Example 23 | Example 24 | Example 25 | Example 26 | Example 27 |
|---|---|---|---|---|---|
| Tableting pressure (ton/punch) | 0.70 | 1.20 | 0.80 | 0.85 | 0.50 |
| Hardness (N) | 51 | 58 | 54 | 54 | 50 |
| Tablet strength (N/cm²) | 160 | 173 | 169 | 170 | 157 |
| Disintegration time (seconds) | 22 | 25 | 19 | 19 | 22 |
| Tableting pressure (ton/punch) | 1.40 | 1.80 | 1.40 | 1.25 | 0.90 |
| Hardness (N) | 83 | 80 | 85 | 82 | 85 |
| Tablet strength (N/cm²) | 275 | 247 | 277 | 270 | 284 |
| Hardness/weights (N/mg) | 0.277 | 0.267 | 0.283 | 0.273 | 0.283 |
| Disintegration time (seconds) | 24 | 27 | 20 | 18 | 23 |

### Experimental Example 6

Orally rapidly disintegrating tablets comprising avanafil (i.e., an active ingredient, chemical name: (S)-2-[2-hydroxymethyl-1-pyrrolidinyl-4-(3-chloro-4-methoxybenzylamino)-5-[N-(2-pyrimidinylmethyl)carbamoyl]pyrimidine), D-mannitol (i.e., an excipient) and HPMCAS/HF (i.e., a water-insoluble polymer) were obtained in accordance with Examples 28 to 30 below. Formulating components and contained amounts of the orally rapidly disintegrating tablets as well as each tablet property are shown in the following Table 11 and Table 12, respectively.

### Experimental Example 7

Orally rapidly disintegrating tablets comprising imidapril hydrochloride, D-mannitol (i.e., an excipient), HPMCAS/HF or EC#10 (i.e., a water-insoluble polymer) were obtained in accordance with Examples 31 to 34 below. Formulating components and contained amounts of the orally rapidly disintegrating tablets as well as each tablet property are shown in the following Table 13 and Table 14.

### Experimental Example 8

Each tablet obtained in Examples 28 to 34 was stored for one month under the condition of 25°C/75% RH, and then a hardness and a disintegration time of tablet in the oral cavity thereof were measured (see Table 12 and Table 14). As the result, any delays of disintegration times in the oral cavity were not observed and decreases in the hardness were moderate in each tablet. The result showed that the orally rapidly disintegrating tablet of the present invention can maintain a preferable disintegrating property as the orally rapidly disintegrating tablet and a sufficient hardness for handling through the period exposed in the atmosphere in an automatic tablet packaging machine after the release of tablet from blister package as well as the period from one dose packaging to dosing.

**[Table 11]**

| Components | | Contained amounts (mg) | | |
|---|---|---|---|---|
| | | Example 28 | Example 29 | Example 30 |
| Drug granule | Avanafil | 100 | 100 | 100 |
| | D-Mannitol | 179.5 | 179.5 | 165.5 |
| | HPMCAS/HF | 10.5 | 10.5 | 10.5 |
| | Subtotal | 290 | 290 | 276 |
| Fumaric acid granule | Fumaric acid | 40.0 | 40.0 | 40.0 |
| | Ethyl cellulose | 3.0 | 3.0 | 3.0 |
| | Calcium stearate | 3.0 | 3.0 | 3.0 |
| | Subtotal | 46.0 | 46.0 | 46.0 |
| External additive parts | Croscarmellose sodium | 7.0 | **---** | --- |
| | Crospovidone | --- | 3.5 | --- |
| | Carboxymethylcellulose | --- | --- | 17.5 |
| | Aspartame | | 3.5 | 3.5 |
| | Magnesium stearate | 7.0 | 7.0 | 7.0 |
| | Subtotal | 17.5 | 14.0 | 28.0 |
| Total | | 353.5 | 350.0 | 350.0 |

**[Table 12]**

| | | Example 28 | Example 29 | Example 30 |
|---|---|---|---|---|
| Disintegration time in oral cavity (seconds) | Tablet before humidified storage | 23 | 25 | 26 |
| | Tablet after humidified storage | 26 | 26 | 32 |
| Hardness of tablet (N) | Tablet before humidified storage | 53 | 52 | 52 |
| | Tablet after humidified storage | 42 | 46 | 40 |
| Tablet strength (N/cm²) | Tablet before humidified storage | 146 | 141 | 143 |
| | Tablet after humidified storage | 115 | 125 | 109 |

**[Table 13]**

| Components | Contained amounts of each component (mg) | | | |
|---|---|---|---|---|
| | Example 31 | Example 32 | Example 33 | Example 34 |
| Imidapril hydrochloride | 5.0 | 5.0 | 5.0 | 5.0 |
| D-Mannitol | 146.2 | 148.6 | 146.2 | 145.2 |
| HPMCAS/HF | 4.8 | 1.6 | - | - |
| EC# 10 | - | - | 4.8 | 4.8 |
| Croscarmellose sodium | 3.2 | 3.2 | 3.2 | 3.2 |
| Magnesium stearate | 1.6 | - | 1.6 | - |
| Sodium stearylfumarate | - | 1.6 | - | 1.6 |
| Total | 160.8 | 160 | 160.8 | 160 |

**[Table 14]**

| | | Example 31 | Example 32 | Example 33 | Example 34 |
|---|---|---|---|---|---|
| Disintegration time in oral cavity (seconds) | Tablet before humidified storage | 27 | 13 | 24 | 21 |
| | Tablet after humidified storage | 23 | 13 | 22 | 20 |
| Hardness of tablet (N) | Tablet before humidified storage | 47 | 41 | 42 | 46 |
| | Tablet after humidified storage | 39 | 41 | 36 | 50 |
| Tablet strength (N/cm²) | Tablet before humidified storage | 191 | 169 | 173 | 189 |
| | Tablet after humidified storage | 157 | 169 | 147 | 203 |

### Example 1

(1) After granulation with adding aqueous 80% (W/W) ethanol solution (5 parts by weight) to a mixture of D-mannitol (97 parts by weight) sieved through Japanese Pharmacopoeia 22 mesh sieve (Opening: 710 µm) and hydroxypropylmethylcellulose acetate succinate (grade; HF; 3 parts by weight), the mixture was dried at 50°C for 2 hours in a ventilatory box drying machine to give a granulated granule.

(2) The granulated granule of the above (1) (283.5 parts by weight), crospovidone (15 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 750 kg/punch) to be 300 mg per a tablet to give an orally rapidly disintegrating tablet (hardness: 56N, disintegration time in the oral cavity: 17 seconds).

(3) An orally rapidly disintegrating tablet (weight: 300 mg, hardness: 84N, disintegration time in the oral cavity: 15 seconds) was obtained in the similar manner to the above (2) except for a tableting pressure of 1250 kg/punch.

### Example 2

(1) After granulation with adding aqueous 80% (W/W) ethanol solution (5 parts by weight) to a mixture of erythritol (90 parts by weight) sieved through Japanese Pharmacopoeia 60 mesh sieve (Opening: 250 µm) and hydroxypropylmethylcellulose acetate succinate (grade; HF; 10 parts by weight), the mixture was dried at 50°C for 2 hours in a ventilatory box drying machine to give a granulated granule.
(2) The granulated granule of the above (1) (283.5 parts by weight), crospovidone (15 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 1000 kg/punch) to be 300 mg per a tablet to give an orally rapidly disintegrating tablet (hardness: 51N, disintegration time in the oral cavity: 15 seconds).

### Example 3

### (1) Preparation of Granule

After granulation with adding aqueous 80% (W/W) ethanol solution (5 parts by weight) to a mixture of xylitol (90 parts by weight) which was grinded by using an agate die and then sieved through Japanese Pharmacopoeia 60 mesh sieve (Opening: 250 µm), and hydroxypropylmethylcellulose acetate succinate (grade; HF; 10 parts by weight), the mixture was dried at 50°C for 2 hours in a ventilatory box drying machine to give a granulated granule.

### (2) Preparation of Orally Rapidly Disintegrating Tablet

The granulated granule of the above (1) (283.5 parts by weight), crospovidone (15 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 2000 kg/punch) to be 300 mg per a tablet to give an orally rapidly disintegrating tablet (hardness: 44N, disintegration time in the oral cavity: 42 seconds).

### Example 4

(1) After granulation with adding aqueous 80% (W/W) ethanol solution (5 parts by weight) to a mixture of D-mannitol (97 parts by weight) sieved through Japanese Pharmacopoeia 22 mesh sieve (Opening: 710 µm) and hydroxypropylmethylcellulose acetate succinate (grade; MF; 3 parts by weight), the mixture was dried at 50°C for 2 hours in a ventilatory box drying machine to give a granulated granule.

(2) The granulated granule of the above (1) (283.5 parts by weight), crospovidone (15 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 700 kg/punch) to be 300 mg per a tablet to give an orally rapidly disintegrating tablet (hardness: 60N, disintegration time in the oral cavity: 15 seconds).

(3) An orally rapidly disintegrating tablet (weight: 300 mg, hardness: 87N, disintegration time in the oral cavity: 17 seconds) was obtained in the similar manner to the above (2) except for a tableting pressure of 1100 kg/punch.

### Example 5

(1) After granulation with adding aqueous 80% (W/W) ethanol solution (5 parts by weight) to a mixture of D-mannitol (97 parts by weight) sieved through Japanese Pharmacopoeia 22 mesh sieve (Opening: 710 µm) and hydroxypropylmethylcellulose acetate succinate (grade; LF; 3 parts by weight), the mixture was dried at 50°C for 2 hours in a ventilatory box drying machine to give a granulated granule.

(2) The granulated granule of the above (1) (283.5 parts by weight), crospovidone (15 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 850 kg/punch) to be 300 mg per a tablet to give an orally rapidly disintegrating tablet (hardness: 54N, disintegration time in the oral cavity: 19 seconds).

(3) An orally rapidly disintegrating tablet (weight: 300 mg, hardness: 82N, disintegration time in the oral cavity: 22 seconds) was obtained in the similar manner to the above (2) except for a tableting pressure of 1350 kg/punch.

### Example 6

(1) After granulation with adding aqueous 80% (W/W) ethanol solution (5 parts by weight) to a mixture of D-mannitol (97 parts by weight) sieved through Japanese Pharmacopoeia 22 mesh sieve (Opening: 710 µm) and ethylcellulose (3 parts by weight), the mixture was dried at 50°C for 2 hours in a ventilatory box drying machine to give a granulated granule.

(2) The granulated granule of the above (1) (283.5 parts by weight), crospovidone (15 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 900 kg/punch) to be 300 mg per a tablet to give an orally rapidly disintegrating tablet (hardness: 53N, disintegration time in the oral cavity: 18 seconds).

(3) An orally rapidly disintegrating tablet (weight: 300 mg, hardness: 80N, disintegration time in the oral cavity: 16 seconds) was obtained in the similar manner to the above (2) except for a tableting pressure of 1400 kg/punch.

### Example 7

(1) After granulation with adding aqueous 80% (W/W) ethanol solution (5 parts by weight) to a mixture of D-mannitol (97 parts by weight) sieved through Japanese Pharmacopoeia 22 mesh sieve (Opening: 710 µm) and hydroxypropylmethylcellulose phthalate (grade; HP-50; 3 parts by weight) grinded by an agate die, the mixture was dried at 50°C for 2 hours in a ventilatory box drying machine to give a granulated granule.

(2) The granulated granule of the above (1) (283.5 parts by weight), crospovidone (15 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 900 kg/punch) to be 300 mg per a tablet to give an orally rapidly disintegrating tablet (hardness: 50N, disintegration time in the oral cavity: 18 seconds).

(3) An orally rapidly disintegrating tablet (weight: 300 mg, hardness: 80N, disintegration time in the oral cavity: 17 seconds) was obtained in the similar manner to the above (2) except for a tableting pressure of 1500 kg/punch.

### Example 8

(1) After granulation with adding aqueous 80% (W/W) ethanol solution (5 parts by weight) to a mixture of D-mannitol (97 parts by weight) sieved through Japanese Pharmacopoeia 22 mesh sieve (Opening: 710 µm) and amino methacrylate copolymer (Eudragit RSPO; 3 parts by weight), the mixture was dried at 50°C for 2 hours in a ventilatory box drying machine to give a granulated granule.

(2) The granulated granule of the above (1) (283.5 parts by weight), crospovidone (15 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 900 kg/punch) to be 300 mg per a tablet to give an orally rapidly disintegrating tablet (hardness: 56N, disintegration time in the oral cavity: 17 seconds).

(3) An orally rapidly disintegrating tablet (weight: 300 mg, hardness: 86N, disintegration time in the oral cavity: 18 seconds) was obtained in the similar manner to the above (2) except for a tableting pressure of 1500 kg/punch.

### Example 9

(1) After granulation with adding aqueous 80% (W/W) ethanol solution (5 parts by weight) to a mixture of D-mannitol (97 parts by weight) sieved through Japanese Pharmacopoeia 22 mesh sieve (Opening: 710 µm) and amino methacrylate copolymer (Eudragit RLPO; 3 parts by weight), the mixture was dried at 50°C for 2 hours in a ventilatory box drying machine to give a granulated granule.

(2) The granulated granule of the above (1) (283.5 parts by weight), crospovidone (15 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 800 kg/punch) to be 300 mg per a tablet to give an orally rapidly disintegrating tablet (hardness: 53N, disintegration time in the oral cavity: 16 seconds).

(3) An orally rapidly disintegrating tablet (weight: 300 mg, hardness: 84N, disintegration time in the oral cavity: 16 seconds) was obtained in the similar manner to the above (2) except for a tableting pressure of 1250 kg/punch.

### Example 10

(1) After granulation with adding aqueous 80% (W/W) ethanol solution (5 parts by weight) to a mixture of D-mannitol (97 parts by weight) sieved through Japanese Pharmacopoeia 22 mesh sieve (Opening: 710 µm) and methacrylic acid copolymer (Eudragit L100; 3 parts by weight), the mixture was dried at 50°C for 2 hours in a ventilatory box drying machine to give a granulated granule.

(2) The granulated granule of the above (1) (283.5 parts by weight), crospovidone (15 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 800 kg/punch) to be 300 mg per a tablet to give an orally rapidly disintegrating tablet (hardness: 51N, disintegration time in the oral cavity: 16 seconds).

(3) An orally rapidly disintegrating tablet (weight: 300 mg, hardness: 81N, disintegration time in the oral cavity: 19 seconds) was obtained in the similar manner to the above (2) except for a tableting pressure of 1250 kg/punch.

### Example 11

(1) After granulation with adding aqueous 80% (W/W) ethanol solution (5 parts by weight) to a mixture of D-mannitol (97 parts by weight) sieved through Japanese Pharmacopoeia 22 mesh sieve (Opening: 710 µm) and methacrylic acid copolymer (Eudragit S100; 3 parts by weight), the mixture was dried at 50°C for 2 hours in a ventilatory box drying machine to give a granulated granule.

(2) The granulated granule of the above (1) (283.5 parts by weight), crospovidone (15 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 1000 kg/punch) to be 300 mg per a tablet to give an orally rapidly disintegrating tablet (hardness: 54N, disintegration time in the oral cavity: 19 seconds).

(3) An orally rapidly disintegrating tablet (weight: 300 mg, hardness: 84N, disintegration time in the oral cavity: 19 seconds) was obtained in the similar manner to the above (2) except for a tableting pressure of 1900 kg/punch.

### Example 12

(1) After granulation with adding aqueous 80% (W/W) ethanol solution (5 parts by weight) to a mixture of D-mannitol (97 parts by weight) sieved through Japanese Pharmacopoeia 22 mesh sieve (Opening: 710 µm) and carboxymethylethylcellulose (3 parts by weight), the mixture was dried at 50°C for 2 hours in a ventilatory box drying machine to give a granulated granule.

(2) The granulated granule of the above (1) (283.5 parts by weight), crospovidone (15 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 1000 kg/punch) to be 300 mg per a tablet to give an orally rapidly disintegrating tablet (weight: 300 mg, hardness: 54N, disintegration time in the oral cavity: 19 seconds).

(3) An orally rapidly disintegrating tablet (weight: 300 mg, hardness: 84N, disintegration time in the oral cavity: 19 seconds) was obtained in the similar manner to the above (2) except for a tableting pressure of 1900 kg/punch.

### Example 13

(1) Imidapril hydrochloride (1.8 parts by weight) and D-mannitol (95 parts by weight) sieved through Japanese Pharmacopoeia 22 mesh sieve (Opening: 710 µm) were placed in a fluid-bed granulator (manufactured by Powrex Corporation, MP-01/03), and the mixture was granulated with spraying aqueous 80% (W/W) ethanol solution (39.7 parts by weight) of 8% hydroxypropylmethylcellulose acetate succinate (grade; HF) at a inlet air temperature of 60°C over about 30 minutes. The granule was dried to the temperature of 40°C or above to give a granulated granule.

(2) The granulated granule of the above (1) (283.5 parts by weight), crospovidone (15 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 800 kg/punch) to be 300 mg per a tablet to give an orally rapidly disintegrating tablet (weight: 300 mg, hardness: 55N, disintegration time in the oral cavity: 18 seconds).

(3) An orally rapidly disintegrating tablet (weight: 300 mg, hardness: 86N, disintegration time in the oral cavity: 20 seconds) was obtained in the similar manner to the above (2) except for a tableting pressure of 1400 kg/punch.

### Example 14

(1) Imidapril hydrochloride (1.8 parts by weight) and D-mannitol (95 parts by weight) sieved through Japanese Pharmacopoeia 22 mesh sieve (Opening: 710 µm) were placed in a fluid-bed granulator (manufactured by Powrex Corporation, MP-01/03), and the mixture was granulated with spraying aqueous 80% (W/W) ethanol solution (39.7 parts by weight) of 8% hydroxypropylmethylcellulose acetate succinate (grade; MF) at a inlet air temperature of 60°C over about 30 minutes. The granule was dried to the temperature of 40°C or above to give a granulated granule.

(2) The granulated granule of the above (1) (283.5 parts by weight), crospovidone (15 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 800 kg/punch) to be 300 mg per a tablet to give an orally rapidly disintegrating tablet (weight: 300 mg, hardness: 59N, disintegration time in the oral cavity: 22 seconds).

(3) An orally rapidly disintegrating tablet (weight: 300 mg, hardness: 87N, disintegration time in the oral cavity: 22 seconds) was obtained in the similar manner to the above (2) except for a tableting pressure of 1400 kg/punch.

### Example 15

(1) Imidapril hydrochloride (1.8 parts by weight) and D-mannitol (95 parts by weight) sieved through Japanese Pharmacopoeia 22 mesh sieve (Opening: 710 µm) were placed in a fluid-bed granulator (manufactured by Powrex Corporation, MP-01/03), and the mixture was granulated with spraying aqueous 80% (W/W) ethanol solution (39.7 parts by weight) of 8% hydroxypropylmethylcellulose acetate succinate (grade; LF) at a inlet air temperature of 60°C over about 30 minutes. The granule was dried to the temperature of 40°C or above to give the granulated granule.

(2) The granulated granule of the above (1) (283.5 parts by weight), crospovidone (15 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 900 kg/punch) to be 300 mg per a tablet to give an orally rapidly disintegrating tablet (hardness: 51N, disintegration time in the oral cavity: 19 seconds).

(3) An orally rapidly disintegrating tablet (weight: 300 mg, hardness: 80N, disintegration time in the oral cavity: 26 seconds) was obtained in the similar manner to the above (2) except for a tableting pressure of 1600 kg/punch.

### Example 16

(1) Imidapril hydrochloride (1.8 parts by weight) and D-mannitol (95 parts by weight) sieved through Japanese Pharmacopoeia 22 mesh sieve (Opening: 710 µm) were placed in a fluid-bed granulator (manufactured by Powrex Corporation, MP-01/03), and the mixture was granulated with spraying aqueous 80% (W/W) ethanol solution (39.7 parts by weight) of 8% hydroxypropylmethylcellulose phthalate (grade; HP-50) at a inlet air temperature of 60°C over about 30 minutes. The granule was dried to the temperature of 40°C or above to give a granulated granule.

(2) The granulated granule of the above (1) (283.5 parts by weight), crospovidone (15 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 800 kg/punch) to be 300 mg per a tablet to give an orally rapidly disintegrating tablet (hardness: 53N, disintegration time in the oral cavity: 19 seconds).

(3) An orally rapidly disintegrating tablet (weight: 300 mg, hardness: 84N, disintegration time in the oral cavity: 23 seconds) was obtained in the similar manner to the above (2) except for a tableting pressure of 1400 kg/punch.

### Example 17

(1) Imidapril hydrochloride (1.8 parts by weight) and D-mannitol (95 parts by weight) sieved through Japanese Pharmacopoeia 22 mesh sieve (Opening: 710 µm) were placed in a fluid-bed granulator (manufactured by Powrex Corporation, MP-01/03), and the mixture was granulated with spraying aqueous 80% (W/W) ethanol solution (39.7 parts by weight) of 8% amino methacrylate copolymer (Eudragit RSPO) at a inlet air temperature of 60°C over about 30 minutes. The granule was dried to the temperature of 40°C or above to give a granulated granule.

(2) The granulated granule of the above (1) (283.5 parts by weight), crospovidone (15 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 550 kg/punch) to be 300 mg per a tablet to give an orally rapidly disintegrating tablet (hardness: 57N, disintegration time in the oral cavity: 23 seconds).

(3) An orally rapidly disintegrating tablet (weight: 300 mg, hardness: 82N, disintegration time in the oral cavity: 20 seconds) was obtained in the similar manner to the above (2) except for a tableting pressure of 800 kg/punch.

### Example 18

(1) Imidapril hydrochloride (1.8 parts by weight) and D-mannitol (95 parts by weight) sieved through Japanese Pharmacopoeia 22 mesh sieve (Opening: 710 µm) were placed in a fluid-bed granulator (manufactured by Powrex Corporation, MP-01/03), and the mixture was granulated with spraying aqueous 80% (W/W) ethanol solution (39.7 parts by weight) of 8% carboxymethylethylcellulose at a inlet air temperature of 60°C over about 30 minutes. The granule was dried to the temperature of 40°C or above to give a granulated granule.

(2) The granulated granule of the above (1) (283.5 parts by weight), crospovidone (15 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 600 kg/punch) to be 300 mg per a tablet to give an orally rapidly disintegrating tablet (hardness: 56N, disintegration time in the oral cavity: 26 seconds).

(3) An orally rapidly disintegrating tablet (weight: 300 mg, hardness: 81N, disintegration time in the oral cavity: 27 seconds) was obtained in the similar manner to the above (2) except for a tableting pressure of 950 kg/punch.

### Example 19

(1) The granulated granule of Example 14(1) (283.5 parts by weight), carboxymethylcellulose (15 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 800 kg/punch) to be 300 mg per a tablet to give an orally rapidly disintegrating tablet (hardness: 50N, disintegration time in the oral cavity: 23 seconds).

(2) An orally rapidly disintegrating tablet (weight: 300 mg, hardness: 80N, disintegration time in the oral cavity: 25 seconds) was obtained in the similar manner to the above (1) except for a tableting pressure of 1400 kg/punch.

### Example 20

(1) The granulated granule of Example 14(1) (283.5 parts by weight), croscarmellose sodium (6 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 800 kg/punch) to be 291 mg per a tablet to give an orally rapidly disintegrating tablet (hardness: 52N, disintegration time in the oral cavity: 20 seconds).

(2) An orally rapidly disintegrating tablet (weight: 291mg, hardness: 81N, disintegration time in the oral cavity: 23 seconds) was obtained in the similar manner to the above (1) except for a tableting pressure of 1450 kg/punch.

### Example 21

(1) The granulated granule of Example 14(1) (283.5 parts by weight), carmellose calcium (15 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 800 kg/punch) to be 300 mg per a tablet to give an orally rapidly disintegrating tablet (weight: 300 mg, hardness: 50N, disintegration time in the oral cavity: 28 seconds).

(2) An orally rapidly disintegrating tablet (weight: 300 mg, hardness: 83N, disintegration time in the oral cavity: 33 seconds) was obtained in the similar manner to the above (1) except for a tableting pressure of 1450 kg/punch.

### Example 22

(1) The granulated granule of Example 14(1) (283.5 parts by weight), carboxymethyl starch sodium (9 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 850 kg/punch) to be 294 mg per a tablet to give an orally rapidly disintegrating tablet (hardness: 53N, disintegration time in the oral cavity: 29 seconds).

(2) An orally rapidly disintegrating tablet (weight: 294mg, hardness: 81N, disintegration time in the oral cavity: 35 seconds) was obtained in the similar manner to the above (1) except for a tableting pressure of 1500 kg/punch.

### Example 23

(1) Bisoprolol fumarate (1.8 parts by weight) and D-mannitol (95 parts by weight) sieved through Japanese Pharmacopoeia 22 mesh sieve (Opening: 710 µm) were placed in a fluid-bed granulator (manufactured by Powrex Corporation, MP-01/03), and the mixture was granulated with spraying 8% hydroxypropylmethylcellulose acetate succinate (grade; HF) aqueous 80% (W/W) ethanol solution (39.7 parts by weight) at a inlet air temperature of 60°C over about 30 minutes. The granule was dried to the temperature of 40°C or above to give a granulated granule.

(2) The granulated granule of the above (1) (283.5 parts by weight), crospovidone (15 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 700 kg/punch) to be 300 mg per a tablet to give an orally rapidly disintegrating tablet (hardness: 51N, disintegration time in the oral cavity: 22 seconds).

(3) An orally rapidly disintegrating tablet (weight: 300 mg, hardness: 83N, disintegration time in the oral cavity: 24 seconds) was obtained in the similar manner to the above (2) except for a tableting pressure of 1400 kg/punch.

### Example 24

(1) Bisoprolol fumarate (2 parts by weight), D-mannitol (88 parts by weight) sieved through Japanese Pharmacopoeia 22 mesh sieve (Opening: 710 µm) and methacrylic acid copolymer L (Eudragit L; 10 parts by weight) were placed in a fluid-bed granulator (manufactured by Powrex Corporation, MP-01/03), and the mixture was granulated with spraying purified water (54.1 parts by weight) at a inlet air temperature of 60°C over about 25 minutes, and then the granule was dried to the temperature of 40°C or above to give a granulated granule.

(2) The granulated granule of the above (1) (295.5 parts by weight), carboxymethylcellulose (33 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 700 kg/punch) to be 300 mg per a tablet to give an orally rapidly disintegrating tablet (hardness: 58N, disintegration time in the oral cavity: 25 seconds).

(3) An orally rapidly disintegrating tablet (weight: 330 mg, hardness: 80N, disintegration time in the oral cavity: 27 seconds) was obtained in the similar manner to the above (2) except for a tableting pressure of 1800 kg/punch.

### Example 25

(1) D-Mannitol (97 parts by weight) sieved through Japanese Pharmacopoeia 22 mesh sieve (Opening: 710 µm) was placed in a fluid-bed granulator (manufactured by Powrex Corporation, MP-01/03), and granulated with spraying aqueous 80% (W/W) ethanol solution (37.5 parts by weight) of 8% hydroxypropylmethylcellulose acetate succinate (grade; HF) at a inlet air temperature of 60°C over about 30 minutes, and then the granule was dried to the temperature of 40°C or above to give a granulated granule.

(2) The granulated granule of the above (1) (278.5 parts by weight), nicergoline (5 parts by weight), crospovidone (15 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 800 kg/punch) to be 300 mg per a tablet to give an orally rapidly disintegrating tablet (hardness: 54N, disintegration time in the oral cavity: 19 seconds).

(3) An orally rapidly disintegrating tablet (weight: 300 mg, hardness: 85N, disintegration time in the oral cavity: 20 seconds) was obtained in the similar manner to the above (2) except for a tableting pressure of 1400 kg/punch.

### Example 26

(1) Taltirelin hydrate (2 parts by weight), D-mannitol (88 parts by weight) sieved through Japanese Pharmacopoeia 22 mesh sieve (Opening: 710 µm) and methacrylic acid copolymer S (Eudragit S; 10 parts by weight) were placed in a high speed mixer granulator (manufactured by Fukae Powtec., an ultracompact high speed mixer), and pre-mixed for one minute. After granulation for about 3 minutes with adding purified water (9.3 parts by weight) to the mixture, the granule was dried at 50°C for 2 hours in a ventilatory box drying machine to give a granulated granule.

(2) The granulated granule of the above (1) (268.5 parts by weight), carboxymethylcellulose (30 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 850 kg/punch) to be 300 mg per a tablet to give an orally rapidly disintegrating tablet (hardness: 54N, disintegration time in the oral cavity: 19 seconds).

(3) An orally rapidly disintegrating tablet (weight: 300 mg, hardness: 82N, disintegration time in the oral cavity: 18 seconds) was obtained in the similar manner to the above (2) except for a tableting pressure of 1250 kg/punch.

### Example 27

(1) Allopurinol (35 parts by weight) and D-mannitol (62 parts by weight) sieved through Japanese Pharmacopoeia 22 mesh sieve (Opening: 710 µm) were placed in a fluid-bed granulator (manufactured by Powrex Corporation, MP-01/03), and the mixture was granulated with spraying aqueous 80% (W/W) ethanol solution (39.7 parts by weight) of 8% hydroxypropylmethylcellulose acetate succinate (grade; HF) at a inlet air temperature of 60°C over about 15 minutes, and then the granule was dried to the temperature of 40°C or above to give a granulated granule.

(2) The granulated granule of the above (1) (283.5 parts by weight), crospovidone (15 parts by weight) and magnesium stearate (1.5 parts by weight) were mixed to give a granule for tableting. The granule for tableting was compressed by Compaction Analyzer (manufactured by Kikusui Seisakusho Ltd., punch: diameter 10 mm, tableting pressure: 500 kg/punch) to be 300 mg per a tablet to give an orally rapidly disintegrating tablet (hardness: 50N, disintegration time in the oral cavity: 22 seconds).

(3) An orally rapidly disintegrating tablet (weight: 300 mg, hardness: 85N, disintegration time in the oral cavity: 23 seconds) was obtained in the similar manner to the above (2) except for a tableting pressure of 900 kg/punch.

### Example 28

(1) Avanafil (34.5 parts by weight) and D-mannitol (61.9 parts by weight) sieved through Japanese Pharmacopoeia 22 mesh sieve (Opening: 710 µm) were placed in a fluid-bed granulator (manufactured by Powrex Corporation, MP-01/03), and the mixture was granulated with spraying a dispersion solution of hydroxypropylmethylcellulose acetate succinate (grade; HF; 3.6 parts by weight) in purified water (53.1 parts by weight) at a inlet air temperature of 50°C over about 25 minutes. The granule was dried to the temperature of 37°C or above to give a granule of an active pharmaceutical ingredient.

(2) Fumaric acid (87 parts by weight) was placed in a fluid-bed granulator (manufactured by Powrex Corporation, MP-01/03), and granulated with spraying a dispersion solution of ethylcellulose (6.5 parts by weight) and calcium stearate (6.5 parts by weight) in aqueous 80% (W/W) ethanol solution (169.5 parts by weight) at a inlet air temperature of 60°C over about 180 minutes. The granule was dried to the temperature of 45°C or above to give a fumaric acid granule.

(3) The granule of an active pharmaceutical ingredient of the above (1) (290 parts by weight), the fumaric acid granule of the above (2) (46 parts by weight), croscarboxymethylcellulose sodium (7 parts by weight), aspartame (3.5 parts by weight) and magnesium stearate (7 parts by weight) were mixed to give a granule for tableting. The granule for tableting was compressed by a rotary tableting machine (manufactured by Kikusui Seisakusho Ltd., COLLECT 12HUK, punch: diameter 10 mm, tableting pressure: 650 kg/punch) to be 353.5 mg per a tablet to give an orally rapidly disintegrating tablet (hardness: 53N, disintegration time in the oral cavity: 23 seconds).

A hardness and a disintegration time in the oral cavity of the above tablet were measured after storage of one month under 25°C/75% RH. As the result, the hardness was 42N, and the disintegration time in the oral cavity was 26 seconds.

### Example 29

The granule of an active pharmaceutical ingredient of Example 28(1) (290 parts by weight), the fumaric acid granule of Example 28(2) (46 parts by weight), crospovidone (3.5 parts by weight), aspartame (3.5 parts by weight) and magnesium stearate (7 parts by weight) were mixed to give a granule for tableting. The granule for tableting was compressed by a rotary tableting machine (manufactured by Kikusui Seisakusho Ltd., COLLECT 12HUK, punch: diameter 10 mm, tableting pressure: 600 kg/punch) to be 350 mg per a tablet to give an orally rapidly disintegrating tablet (hardness: 52N, disintegration time in the oral cavity: 25 seconds).

A hardness and a disintegration time in the oral cavity of the above tablet were measured after storage of one month under 25°C/75% RH. As the result, the hardness was 46N, and the disintegration time in the oral cavity was 26 seconds.

### Example 30

(1) Avanafil (36.2 parts by weight) and D-mannitol (60 parts by weight) sieved through Japanese Pharmacopoeia 22 mesh sieve (Opening: 710 µm) were placed in a fluid-bed granulator (manufactured by Powrex Corporation, MP-01/03), and the mixture was granulated with spraying a dispersion solution of hydroxypropylmethylcellulose acetate succinate (grade; HF; 3.8 parts by weight) in purified water (55.7 parts by weight) at a inlet air temperature of 50°C over about 25 minutes. The granule was dried to the temperature of 37°C or above to give a granule of an active pharmaceutical ingredient.

(2) The granule of an active pharmaceutical ingredient of the above (1) (276 parts by weight), the fumaric acid granule of Example 28(2) (46 parts by weight), carboxymethylcellulose (17.5 parts by weight), aspartame (3.5 parts by weight) and magnesium stearate (7 parts by weight) were mixed to give a granule for tableting. The granule for tableting was compressed by a rotary tableting machine (manufactured by Kikusui Seisakusho Ltd., COLLECT 12HUK, punch: diameter 10 mm, tableting pressure: 750 kg/punch) to be 350 mg per a tablet to give an orally rapidly disintegrating tablet (hardness: 52N, disintegration time in the oral cavity: 26 seconds).

A hardness and a disintegration time in the oral cavity of the above tablet were measured after storage of one month under 25°C/75% RH. As the result, the hardness was 40N, and the disintegration time in the oral cavity was 32 seconds.

### Example 31

(1) Imidapril hydrochloride (3.1 parts by weight) and D-mannitol (90.9 parts by weight) sieved through Japanese Pharmacopoeia 22 mesh sieve (Opening: 710 µm) were placed in a fluid-bed granulator (manufactured by Powrex Corporation, MP-01/03), and granulated with spraying aqueous 80% (W/W) ethanol solution (38.5 parts by weight) of 8% hydroxypropylmethylcellulose acetate succinate (grade; HF) at a inlet air temperature of 60°C over about 30 minutes. The granule was dried to the temperature of 40°C or above to give a granulated granule.

(2) The granulated granule of the above (1) (156 parts by weight), croscarmellose sodium (3.2 parts by weight) and magnesium stearate (1.6 parts by weight) were mixed to give a granule for tableting. The granule for tableting was compressed by a rotary tableting machine (manufactured by Kikusui Seisakusho Ltd., VERA, punch: diameter 7.5 mm, tableting pressure: 500 kg/punch) to be 160.8 mg per a tablet to give an orally rapidly disintegrating tablet (hardness: 47N, disintegration time in the oral cavity: 27 seconds).

A hardness and a disintegration time in the oral cavity of the above tablet were measured after storage of one month under 25°C/75% RH. As the result, the hardness was 39N, and the disintegration time in the oral cavity was 23 seconds.

### Example 32

(1) Imidapril hydrochloride (3.1 parts by weight) and D-mannitol (92.9 parts by weight) sieved through Japanese Pharmacopoeia 22 mesh sieve (Opening: 710 µm) were placed in a fluid-bed granulator (manufactured by Powrex Corporation, MP-01/03), and the mixture was granulated with spraying aqueous 80% (W/W) ethanol solution (38.7 parts by weight) of 2.7% hydroxypropylmethylcellulose acetate succinate (grade; HF) at a inlet air temperature of 50°C over about 15 minutes. The granule was dried to the temperature of 40°C or above to give a granulated granule.

(2) The granulated granule of the above (1) (155.2 parts by weight), croscarmellose sodium (3.2 parts by weight) and magnesium stearate (1.6 parts by weight) were mixed to give a granule for tableting. The granule for tableting was compressed by a rotary tableting machine (manufactured by Kikusui Seisakusho Ltd., VERA, punch: diameter 7.5 mm, tableting pressure: 600 kg/punch) to be 160 mg per a tablet to give an orally rapidly disintegrating tablet (hardness: 41N, disintegration time in the oral cavity: 13 seconds).

A hardness and a disintegration time in the oral cavity of the above tablet were measured after storage of one month under 25°C/75% RH. As the result, the hardness was 41N, and the disintegration time in the oral cavity was 13 seconds.

### Example 33

(1) Imidapril hydrochloride (3.1 parts by weight) and D-mannitol (90.9 parts by weight) sieved through Japanese Pharmacopoeia 22 mesh sieve (Opening: 710 µm) were placed in a fluid-bed granulator (manufactured by Powrex Corporation, MP-01/03), and the mixture was granulated with spraying aqueous 80% (W/W) ethanol solution (38.5 parts by weight) of 8% ethylcellulose at a inlet air temperature of 50°C over about 15 minutes. The granule was dried to the temperature of 40°C or above to give a granulated granule.

(2) The granulated granule of the above (1) (155.2 parts by weight), croscarmellose sodium (3.2 parts by weight) and magnesium stearate (1.6 parts by weight) were mixed to give a granule for tableting. The granule for tableting was compressed by a rotary tableting machine (manufactured by Kikusui Seisakusho Ltd., VERA, punch: diameter 7.5 mm, tableting pressure: 500 kg/punch) to be 160.8 mg per a tablet to give an orally rapidly disintegrating tablet (hardness: 42N, disintegration time in the oral cavity: 24 seconds).

A hardness and a disintegration time in the oral cavity of the above tablet were measured after storage of one month under 25°C/75% RH. As the result, the hardness was 36N, and the disintegration time in the oral cavity was 22 seconds.

### Example 34

(1) Imidapril hydrochloride (3.1 parts by weight) and D-mannitol (90.9 parts by weight) sieved through Japanese Pharmacopoeia 22 mesh sieve (Opening: 710 µm) were placed in a fluid-bed granulator (manufactured by Powrex Corporation, MP-01/03), and the mixture was granulated with spraying aqueous 80% (W/W) ethanol solution (38.7 parts by weight) of 8% ethylcellulose at a inlet air temperature of 50°C over about 15 minutes. The granule was dried to the temperature of 40°C or above to give a granulated granule.

(2) The granulated granule of the above (1) (155.2 parts by weight), croscarmellose sodium (3.2 parts by weight) and sodium stearylfumarate (1.6 parts by weight) were mixed to give a granule for tableting. The granule for tableting was compressed by a rotary tableting machine (manufactured by Kikusui Seisakusho Ltd., VERA, punch: diameter 7.5 mm, tableting pressure: 500 kg/punch) to be 160 mg per a tablet to give an orally rapidly disintegrating tablet (hardness: 46N, disintegration time in the oral cavity: 21 seconds).

A hardness and a disintegration time in the oral cavity of the above tablet were measured after storage of one month under 25°C/75% RH. As the result, the hardness was 50N, and the disintegration time in the oral cavity was 20 seconds.

### Comparative Example 1

(1) After granulation with adding aqueous 80% (W/W) ethanol solution (5 parts by weight) to a mixture of sorbitol (97 parts by weight) sieved through Japanese Pharmacopoeia 60 mesh sieve (Opening: 250 µm) and hydroxypropylmethylcellulose acetate succinate (grade; HF; 3 parts by weight), the granule was dried at 50°C for 2 hours in a ventilatory box drying machine to give a granulated granule.

(2) The granulated granule of the above (1) (283.5 parts by weight), crospovidone (15 parts by weight) and magnesium stearate (1.5 parts by weight) were mixed to prepare a granule for tableting. The granule for tableting was compressed by Compaction Analyzer (manufactured by Kikusui Seisakusho Ltd., punch: diameter 10 mm, tableting pressure: 200 kg/punch) to be 300 mg per a tablet to give a tablet (hardness: 57N, disintegration time in the oral cavity: 129 seconds).

### Comparative Example 2

(1) After granulation with adding aqueous 80% (W/W) ethanol solution (5 parts by weight) to a mixture of purified sucrose (97 parts by weight) which was grinded by an agate die and then sieved through Japanese Pharmacopoeia 60 mesh sieve (Opening: 250 µm) and hydroxypropylmethylcellulose acetate succinate (grade; HF; 3 parts by weight), the mixture was dried at 50°C for 2 hours in a ventilatory box drying machine to give a granulated granule.

(2) The granulated granule of the above (1) (283.5 parts by weight), crospovidone (15 parts by weight) and magnesium stearate (1.5 parts by weight) were mixed to prepare a granule for tableting. The granule for tableting was compressed by Compaction Analyzer (manufactured by Kikusui Seisakusho Ltd., punch: diameter 10 mm, tableting pressure: 950 kg/punch) to be 300 mg per a tablet to give a tablet (hardness: 53N, disintegration time in the oral cavity: 65 seconds).

### Comparative Example 3

(1) After granulation with adding aqueous 80% (W/W) ethanol solution (5 parts by weight) to a mixture of D-mannitol (97 parts by weight) sieved through Japanese Pharmacopoeia 22 mesh sieve (Opening: 710 µm) and hydroxypropylmethylcellulose (grade; TC-5EW; 3 parts by weight), the granule was dried at 50°C for 2 hours in a ventilatory box drying machine to give a granulated granule.

(2) The granulated granule of the above (1) (283.5 parts by weight), crospovidone (15 parts by weight) and magnesium stearate (1.5 parts by weight) were mixed to prapare a granule for tableting. The granule for tableting was compressed by Compaction Analyzer (manufactured by Kikusui Seisakusho Ltd., punch: diameter 10 mm, tableting pressure: 900 kg/punch) to be 300 mg per a tablet to give a tablet (hardness: 52N, disintegration time in the oral cavity: 105 seconds).

### Comparative Example 4

(1) After granulation with adding aqueous 80% (W/W) ethanol solution (5 parts by weight) to a mixture of D-mannitol (97 parts by weight) sieved through Japanese Pharmacopoeia 22 mesh sieve (Opening: 710 µm) and hydroxypropylcellulose (grade; HPC-SL; 3 parts by weight), the mixture was dried at 50°C for 2 hours in a ventilatory box drying machine to give a granulated granule.

(2) The granulated granule of the above (1) (283.5 parts by weight), crospovidone (15 parts by weight) and magnesium stearate (1.5 parts by weight) were mixed to prepare a granule for tableting. The granule for tableting was compressed by Compaction Analyzer (manufactured by Kikusui Seisakusho Ltd., punch: diameter 10 mm, tableting pressure: 850 kg/punch) to be 300 mg per a tablet to give a tablet (hardness: 52N, disintegration time in the oral cavity: 37 seconds).

(3) A tablet (weight: 300 mg, hardness: 81N, disintegration time in the oral cavity: 46 seconds) was obtained in the similar manner to the above (2) except for a tableting pressure of 1350 kg/punch.

### Comparative Example 5

(1) After granulation with adding aqueous 80% (W/W) ethanol solution (5 parts by weight) to a mixture of D-mannitol (97 parts by weight) sieved through Japanese Pharmacopoeia 22 mesh sieve (Opening: 710 µm) and corn starch (3 parts by weight), the mixture was dried at 50°C for 2 hours in a ventilatory box drying machine to give a granulated granule.

(2) The granulated granule of the above (1) (283.5 parts by weight), crospovidone (15 parts by weight) and magnesium stearate (1.5 parts by weight) were mixed to prepare a granule for tableting. The granule for tableting was compressed by Compaction Analyzer (manufactured by Kikusui Seisakusho Ltd., punch: diameter 10 mm, tableting pressure: 2000 kg/punch) to be 300 mg per a tablet to give a tablet (hardness: 57N, disintegration time in the oral cavity: 15 seconds). On the other hand, any tablets having a hardness of tablet of 80N or above could not be prepared even at a tableting pressure of 2000 kg/punch.

### Comparative Example 6

(1) After granulation with adding aqueous 80% (W/W) ethanol solution (5 parts by weight) to a mixture of D-mannitol (97 parts by weight) sieved through Japanese Pharmacopoeia 22 mesh sieve (Opening: 710 µm) and crospovidone (3 parts by weight), the granule was dried at 50°C for 2 hours in a ventilatory box drying machine to give a granulated granule.

(2) The granulated granule of the above (1) (283.5 parts by weight), crospovidone (15 parts by weight) and magnesium stearate (1.5 parts by weight) were mixed to prepare a granule for tableting. The granule for tableting was compressed by Compaction Analyzer (manufactured by Kikusui Seisakusho Ltd., punch: diameter 10 mm, tableting pressure: 1500 kg/punch) to be 300 mg per a tablet to give a tablet (hardness: 52N, disintegration time in the oral cavity: 15 seconds). On the other hand, any tablets having a hardness of tablet of 80N or above could not be obtained even at a tableting pressure of 2000 kg/punch.

### INDUSTRIAL APPLICABILITY

The orally rapidly disintegrating tablet of the present invention can be prepared by conventional tablet manufacturing facilities, and has a sufficient hardness with less possibility for damage during handling in each process including manufacturing, distribution and dispensing. The orally rapidly disintegrating tablet of the present invention also has a feature that any changes of properties by an operation by an automatic tablet packaging machine or by factors such as moisture during storage periods thereafter (including a decrease in a hardness of tablet and a delay of a disintegration time in the oral cavity) are less likely to occur, and therefore, is highly feasible for a pharmaceutical formulation, etc.

The present invention can be summarized by reference to the following embodiments (embs.):
Emb. 1. An orally rapidly disintegrating tablet, which is produced by compressing a mixture comprising (a) an active ingredient; (b) an excipient having good water wettability; (c) a water-insoluble polymer that is well compactible and does not substantially cause a decrease in the water wettability of the excipient; and (d) a disintegrating agent; and wherein a disintegration time in the oral cavity is within 60 seconds.
Emb. 2. The orally rapidly disintegrating tablet of embodiment 1, wherein the excipient having good water wettability is one or more sugar alcohols selected from the group consisting of mannitol, erythritol and xylitol; and the water-insoluble polymer that is well compactible and does not substantially cause a decrease in the water wettability of the excipient is one or more water-insoluble polymers selected from the group consisting of hydroxypropylmethylcellulose acetate succinate, ethylcellulose, hydroxypropylmethylcellulose phthalate, carboxymethylethylcellulose, methacrylic acid copolymer L, methacrylic acid copolymer S and amino methacrylate copolymer.
Emb. 3. The orally rapidly disintegrating tablet of embodiment 1 or 2, wherein the contained amounts of the excipient having good water wettability are 30 to 95 parts by weight; the contained amounts of the water-insoluble polymer that is well compactible and does not substantially cause a decrease in the water wettability of the excipient are 1 to 10 parts by weight; and the contained amounts of the disintegrating agent are 1 to 15 parts by weight, in 100 parts by weight of the tablet.
Emb. 4. The orally rapidly disintegrating tablet of embodiment 3, wherein the tablet strength is 100 to 300N/cm².
Emb. 5. The orally rapidly disintegrating tablet of embodiment 3, wherein the tablet strength is 110 to 300N/cm².
Emb. 6. The orally rapidly disintegrating tablet of embodiment 3, wherein the tablet strength is 120 to 300N/cm².
Emb. 7. The orally rapidly disintegrating tablet of any one of embodiments 4, 5 or 6, wherein the disintegration time in the oral cavity is 5 to 45 seconds.
Emb. 8. The orally rapidly disintegrating tablet of any one of embodiments 4, 5 or 6, wherein the disintegration time in the oral cavity is 5 to 30 seconds.
Emb. 9. The orally rapidly disintegrating tablet of any one of embodiments 4, 5 or 6, wherein the disintegration time in the oral cavity is 5 to 20 seconds.
Emb. 10. The orally rapidly disintegrating tablet of any one of embodiments 4, 5 or 6, wherein the ratio of the hardness to the tablet weights is 0.2N/mg or more.
Emb. 11. The orally rapidly disintegrating tablet of any one of embodiments 4, 5 or 6, wherein the ratio of the hardness to the tablet weights is 0.3N/mg or more.
Emb. 12. The orally rapidly disintegrating tablet of any one of embodiments 4, 5 or 6, wherein the active ingredient is nicergoline, imidapril hydrochloride, bisoprolol fumarate, taltirelin hydrate, allopurinol or avanafil.
Emb. 13. The orally rapidly disintegrating tablet of embodiment 12, wherein the active ingredient is nicergoline, imidapril hydrochloride, bisoprolol fumarate or taltirelin hydrate, and the contained amounts of the active ingredient are 0.1 to 70 parts by weight to 100 parts by weight of the tablet.
Emb. 14. The orally rapidly disintegrating tablet of embodiment 12, wherein the active ingredient is nicergoline, imidapril hydrochloride, bisoprolol fumarate or taltirelin hydrate, and the contained amounts of the active ingredient is 0.5 to 20 parts by weight to 100 parts by weight of the tablet.
Emb. 15. The orally rapidly disintegrating tablet of embodiment 12, wherein the active ingredient is nicergoline, imidapril hydrochloride, bisoprolol fumarate or taltirelin hydrate, and the contained amounts of the active ingredient is 1 to 15 parts by weight to 100 parts by weight of the tablet.
Emb. 16. The orally rapidly disintegrating tablet of embodiment 12, wherein the active ingredient is nicergoline, imidapril hydrochloride, bisoprolol fumarate or taltirelin hydrate, and the contained amounts of the active ingredient is 1 to 10 parts by weight to 100 parts by weight of the tablet.
Emb. 17. The orally rapidly disintegrating tablet of embodiment 12, wherein the active ingredient is allopurinol or avanafil, and the contained amounts of the active ingredient is 10 to 50 parts by weight to 100 parts by weight of the tablet.
Emb. 18. An orally rapidly disintegrating tablet, which is produced by compressing a mixture of (a) an active ingredient selected from the group consisting of nicergoline, imidapril hydrochloride, bisoprolol fumarate, taltirelin hydrate, allopurinol and avanafil; (b) one or more excipients selected from the group consisting of mannitol, erythritol and xylitol; (c) one or more water-insoluble polymers selected from the group consisting of hydroxypropylmethylcellulose acetate succinate, ethylcellulose, hydroxypropylmethylcellulose phthalate, carboxymethylethylcellulose, methacrylic acid copolymer L, methacrylic acid copolymer S and amino methacrylate copolymer; (d) one or more disintegrating agents selected from the group consisting of carboxymethylcellulose calcium, low-substituted hydroxypropylcellulose, carboxymethylcellulose, cross-linked carboxymethylcellulose sodium, crystalline cellulose, corn starch, pregelatinized starch, carboxymethyl starch sodium and cross-linked polyvinylpyrrolidone; and (e) a lubricant, wherein the mixture can contain one or more additives selected from the group consisting of binders, plasticizers, coating agents, deflocculating agents, solubilizers, sweeteners, acidulants, flavoring substances, pH adjusters, solubilizing agents, colorants and flavoring agents; and wherein a disintegration time in the oral cavity is within 60 seconds.
Emb. 19. The orally rapidly disintegrating tablet of embodiment 18, wherein the contained amounts of the excipient are 30 to 95 parts by weight, the contained amounts of the water-insoluble polymer are 1 to 10 parts by weight, the contained amounts of the disintegrating agent are 1 to 15 parts by weight, and the contained amounts of the lubricant are 0.01 to 3 parts by weight, in 100 parts by weight of the tablet.
Emb. 20. The orally rapidly disintegrating tablet of embodiment 19, wherein the tablet strength is 100 to 300N/cm².
Emb. 21. The orally rapidly disintegrating tablet of embodiment 19, wherein the tablet strength is 110 to 300N/cm².
Emb. 22. The orally rapidly disintegrating tablet of embodiment 19, wherein the tablet strength is 120 to 300N/cm².
Emb. 23. The orally rapidly disintegrating tablet of any one of embodiments 20, 21 or 22, wherein the disintegration time in the oral cavity is 5 to 45 seconds.
Emb. 24. The orally rapidly disintegrating tablet of any one of embodiments 20, 21 or 22, wherein the disintegration time in the oral cavity is 5 to 30 seconds.
Emb. 25. The orally rapidly disintegrating tablet of any one of embodiments 20, 21 or 22, wherein the disintegration time in the oral cavity is 5 to 20 seconds.
Emb. 26. The orally rapidly disintegrating tablet of any one of of embodiments 20, 21 or 22, wherein the ratio of the hardness to the tablet weights is 0.2N/mg or more.
Emb. 27. The orally rapidly disintegrating tablet of any one of of embodiments 20, 21 or 22, wherein the ratio of the hardness to the tablet weights is 0.3N/mg or more.
Emb. 28. The orally rapidly disintegrating tablet of in any one of of embodiments 20, 21 or 22, wherein the excipient is mannitol, and the water-insoluble polymer is hydroxypropylmethylcellulose acetate succinate, hydroxypropylmethylcellulose phthalate or carboxymethylethylcellulose.
Emb. 29. A process for preparing an orally rapidly disintegrating tablet wherein a disintegration time in the oral cavity is within 60 seconds, comprising i) preparing a mixture of (a) an active ingredient; (b) an excipient having good water wettability; (c) a water-insoluble polymer that is well compactible and does not substantially cause a decrease in the water wettability of the excipient; and (d) a disintegrating agent, wherein the mixture can contain one or more additives selected from the group consisting of lubricants, binders, plasticizers, coating agents, deflocculating agents, solubilizers, sweeteners, acidulants, flavoring substances, pH adjusters, solubilizing agents, colorants and flavoring agents, followed by ii) compressing the mixture obtained in the above i).
Emb. 30. The process of embodiment 29, wherein the active ingredient is nicergoline, imidapril hydrochloride, bisoprolol fumarate, taltirelin hydrate, allopurinol or avanafil; the excipient having good water wettability is mannitol, erythritol or xylitol; the water-insoluble polymer that is well compactible and does not substantially cause a decrease in the water wettability of the excipient is one or more water-insoluble polymers selected from the group consisting of hydroxypropylmethylcellulose acetate succinate, ethylcellulose, hydroxypropylmethylcellulose phthalate, carboxymethylethylcellulose, methacrylic acid copolymer L, methacrylic acid copolymer S and amino methacrylate copolymer; and the disintegrating agent is carboxymethylcellulose calcium, carboxymethylcellulose, cross-linked carboxymethylcellulose sodium, carboxymethyl starch sodium or cross-linked polyvinylpyrrolidone.
Emb. 31. The process of embodiment 30, wherein the tablet strength of the orally rapidly disintegrating tablet is 100 to 300N/cm².
Emb. 32. The orally rapidly disintegrating tablet of either embodiment 1 or 18, wherein the diameter is 5 to 10 mm, the weight is 100 to 300 mg, and the hardness is 15N to 90N.

## Claims

1. An orally rapidly disintegrating tablet, which is produced by compressing a mixture of
(a) an active ingredient selected from the group consisting of avanafil, nicergoline, imidapril hydrochloride, bisoprolol fumarate, taltirelin hydrate, and allopurinol;
(b) one or more excipients selected from the group consisting of mannitol, erythritol and xylitol;
(c) one or more water-insoluble polymers selected from the group consisting of ethylcellulose and amino methacrylate copolymer;
(d) one or more disintegrating agents selected from the group consisting of carboxymethylcellulose calcium, low-substituted hydroxypropylcellulose, carboxymethylcellulose, cross-linked carboxymethylcellulose sodium, crystalline cellulose, corn starch, pregelatinized starch, carboxymethyl starch sodium and cross-linked polyvinylpyrrolidone; and
(e) a lubricant,
wherein the mixture optionally contains one or more additives selected from the group consisting of a binder, a plasticizer, a coating agent, a deflocculating agent, a solubilizer, a sweetener, an acidulant, a flavoring substance, a pH adjuster, a solubilizing agent, a colorant and a flavoring agent; and wherein a disintegration time in the oral cavity is within 60 seconds.

2. The orally rapidly disintegrating tablet as claimed in claim 1, wherein the contained amounts of the excipient are 30 to 95 parts by weight, the contained amounts of the water-insoluble polymer are 1 to 10 parts by weight, the contained amounts of the disintegrating agent are 1 to 15 parts by weight, and the contained amounts of the lubricant are 0.01 to 3 parts by weight, in 100 parts by weight of the tablet.

3. The orally rapidly disintegrating tablet as claimed in claim 2, wherein the tablet strength is 100 to 300 N/cm², preferably 110 to 300 N/cm² and further more preferably 120 to 300 N/cm².

4. The orally rapidly disintegrating tablet as claimed in claim 3, wherein the disintegration time in the oral cavity is 5 to 45 seconds, more preferably 5 to 30 seconds and particularly preferably 5 to 20 seconds.

5. The orally rapidly disintegrating tablet as claimed in claim 3, wherein the ratio of the hardness to the tablet weights is 0.2N/mg or more, preferably 0.3 N/mg or more.

6. The orally rapidly disintegrating tablet as claimed in claim 3, wherein the excipient is mannitol.

7. An orally rapidly disintegrating tablet, in which its disintegration time in the oral cavity is within 60 seconds, which is produced by compressing a mixture comprising
(a) an active ingredient excluding valdecoxib;
(b) an excipient having good water wettability which is one or more sugar alcohols selected from the group consisting of mannitol, erythritol and xylitol;
(c) a water-insoluble polymer that is well compactible and does not substantially cause a decrease in the water wettability of the excipient which is one or more water-insoluble polymers selected from the group consisting of ethylcellulose and amino methacrylate copolymer; and
(d) a disintegrating agent.

8. The orally rapidly disintegrating tablet as claimed in claim 7, wherein the contained amounts of the excipient having good water wettability are 30 to 95 parts by weight; the contained amounts of the water-insoluble polymer that is well compactible and does not substantially cause a decrease in the water wettability of the excipient are 1 to 10 parts by weight; and the contained amounts of the disintegrating agent are 1 to 15 parts by weight, in 100 parts by weight of the tablet.

9. The orally rapidly disintegrating tablet as claimed in claim 8, wherein the tablet strength is 100 to 300 N/cm², preferably 110 to 300 N/cm² and further more preferably 120 to 300 N/cm².

10. The orally rapidly disintegrating tablet as claimed in claim 9, wherein the disintegration time in the oral cavity is 5 to 45 seconds, more preferably 5 to 30 seconds and particularly preferably 5 to 20 seconds.

11. The orally rapidly disintegrating tablet as claimed in claim 9, wherein the ratio of the hardness to the tablet weights is 0.2N/mg or more, preferably 0.3 N/mg or more.

12. The orally rapidly disintegrating tablet as claimed in claim 9, wherein the active ingredient is avanafil, nicergoline, imidapril hydrochloride, bisoprolol fumarate, taltirelin hydrate or allopurinol.

13. The orally rapidly disintegrating tablet as claimed in claim 12, wherein the active ingredient is nicergoline, imidapril hydrochloride, bisoprolol fumarate or taltirelin hydrate, and the contained amounts of the active ingredient are 0.1 to 70 parts by weight, preferably 0.5 to 20 parts by weight, more preferably 1 to 15 parts by weight and particularly preferably 1 to 10 parts by weight of the tablet.

14. The orally rapidly disintegrating tablet as claimed in claim 12, wherein the active ingredient is avanafil or allopurinol, and the contained amounts of the active ingredient is 10 to 50 parts by weight to 100 parts by weight of the tablet.

15. A process for preparing an orally rapidly disintegrating tablet, wherein a disintegration time in the oral cavity is within 60 seconds, comprising the following steps of
i) preparing a mixture of
(a) an active ingredient excluding valdecoxib;
(b) an excipient having good water wettability which is one or more sugar alcohols selected from the group consisting of mannitol, erythritol and xylitol;
(c) a water-insoluble polymer that is well compactible and does not substantially cause a decrease in the water wettability of the excipient which is one or more water-insoluble polymers selected from the group consisting of ethylcellulose and amino methacrylate copolymer; and
(d) a disintegrating agent; and
wherein the mixture optionally contains one or more additives selected from the group consisting of a lubricant, a binder, a plasticizer, a coating agent, a deflocculating agent, a solubilizer, a sweetener, an acidulant, a flavoring substance, a pH adjuster, a solubilizing agent, a colorant and a flavoring agent, followed by
ii) compressing the mixture obtained in the above i).

16. The process as claimed in claim 15, wherein the active ingredient is avanafil, nicergoline, imidapril hydrochloride, bisoprolol fumarate, taltirelin hydrate or allopurinol; and the disintegrating agent is carboxymethylcellulose calcium, carboxymethylcellulose, cross-linked carboxymethylcellulose sodium, carboxymethyl starch sodium or cross-linked polyvinylpyrrolidone.

17. The process as claimed in claim 16, wherein the tablet strength of the orally rapidly disintegrating tablet is 100 to 300 N/cm².

18. The process as claimed in either claim 1 or 7, wherein the diameter is 5 to 10 mm, the weight is 100 to 300 mg, and the hardness is 15 N to 90 N.
